# EUROPEAN PATENT APPLICATION

(11) **EP 3 639 825 A1**
(43) Date of publication of application: **22.04.2020**
(21) Application number: 18818453.5
(22) Date of filing: 14.06.2018
(51) Int. Cl.: A61K 31/445, A61K 31/55, A61K 38/47, A61P 3/06, A61P 43/00, C12N 9/24

(54) **PHARMACEUTICAL COMBINATION FOR TREATMENT OF FABRY DISEASE AND USE THEREOF**

(30) Priority: 14.06.2017 JP 2017117266
(71) Applicant: Meiji Pharmaceutical University, Tokyo 204-8588 (JP)
(72) Inventor: TSUKIMURA, Takahiro, Kiyose-shi Tokyo 204-8588 (JP); TOGAWA, Tadayasu, Kiyose-shi Tokyo 204-8588 (JP); SAKURABA, Hitoshi, Kiyose-shi Tokyo 204-8588 (JP)
(74) Representative: Schön, Christoph
(86) International application number: PCT/JP2018/022679
(87) International publication number: WO 2018/230628

(57) **Abstract**

The present invention provides a pharmaceutical combination for treatment of Fabry disease and use thereof. The present invention relates to a pharmaceutical combination for treating Fabry disease, and the pharmaceutical combination includes (i) a protein which has mutations in an amino acid sequence of α-N-acetylgalactosaminidase and has α-galactosidase activity and (ii) an active site specific chaperone.

## Description

### Technical Field

The present invention relates to a pharmaceutical combination for treatment of Fabry disease and to use of the pharmaceutical combination.

### Background Art

Fabry disease is an X chromosomal genetic disease in which globotriaosylceramide (Gb3) and globotriaosylsphingosine (Lyso-Gb3) which are substrates are accumulated due to significant decrease in activity of α-galactosidase (α-GAL) which is a lysosomal hydrolase. Recently, as a method for treating Fabry disease, an enzyme replacement therapy is introduced in which recombinant α-GLA (agalsidase alfa and agalsidase beta) is administered.

In Europe, a chaperone therapy is approved in which 1-deoxy galactonojirimycin (also known as Migalstat), which is a substrate analog of α-GAL, is used. In recent years, a clinical research has been carried out in which recombinant α-GAL and 1-deoxy galactonojirimycin were simultaneously administered to a patient of Fabry disease, and it is reported that the administered recombinant α-GAL was stabilized by 1-deoxy galactonojirimycin in the blood and consequently a therapeutic effect of the recombinant α-GAL was enhanced (see Patent Literatures 1 through 3 and Non-patent Literature 1).

Meanwhile, it is reported that the enzyme replacement therapy includes administration of recombinant α-GAL which is deficient in the living body, and therefore an antibody to the recombinant α-GAL is produced and this deteriorates the therapeutic effect (see Non-patent Literature 2).

The present inventors have so far developed an α-N-acetylgalactosaminidase (α-NAGA) mutant as an enzyme formulation that is expected to hardly lead to production of an antibody even when being administered to a patient of Fabry disease (see Patent Literatures 4 and 5 and Non-patent Literature 3). The α-NAGA mutant is an enzyme that has α-GAL activity obtained by altering two amino acid residues into the α-GAL form in a substrate recognition site of NAGA. So far, it has been confirmed that Gb3 and Lyso-Gb3 which are accumulated in organs are degraded in a Fabry disease model mouse to which the α-NAGA mutant has been administered (see Patent Literatures 4 and 5 and Non-patent Literature 3).

### Citation List

### [Patent Literature]

[Patent Literature 1]
   Japanese Patent Application Publication Tokukai No. 2012-107020 (Publication date: June 7, 2012)
[Patent Literature 2]
   Japanese Patent Application Publication Tokukai No. 2012-102128 (Publication date: May 31, 2012)
[Patent Literature 3]
   Published Japanese Translation of PCT International Application Tokuhyo No. 2014-528901 (Publication Date: October 30, 2010)
[Patent Literature 4]
   International Publication No. WO 2007/058381 (Publication Date: May 24, 2007) (Patent No. 4368925)
[Patent Literature 5]
   International Publication No. WO 2008/143354 (Publication Date: November 27, 2008) (Patent No. 5507242)

### [Non-patent Literature]

[Non-patent Literature 1]
   Warnock D et al., PLoS One. 2015, 10:e0134341.
[Non-patent Literature 2]
   Lenders M et al., J Am Soc Nephrol. 2016, 27:256-64.
[Non-patent Literature 3]
   Tajima Y et al., Am J Hum Genet. 2009, 85:569-80.

### Summary of Invention

### Technical Problem

A recombinant enzyme is unstable, and an administered enzyme formulation is therefore gradually denatured in the blood or in the cell. Moreover, due to repetitive administration of the recombinant α-GAL, an antibody to the enzyme is produced, and this may cause deleterious side reaction and deterioration in therapeutic effect of the enzyme. Under the circumstances, an enzyme formulation is demanded which is more stable and has a higher therapeutic effect.

### Solution to Problem

The present inventors searched for a compound that enhances stability and a therapeutic effect on Fabry disease of an α-NAGA mutant by being administered simultaneously with the α-NAGA mutant. Then, the present inventors found that stability of the α-NAGA mutant is enhanced when the α-NAGA mutant is caused to make contact with a certain type of compound, and have thus accomplished the present invention. That is, the present invention encompasses any one of the following aspects:
<1> A pharmaceutical combination for treating Fabry disease, the pharmaceutical combination including:
   a protein which has mutations in an amino acid sequence of α-N-acetylgalactosaminidase and has α-galactosidase activity; and
   an active site specific chaperone,
   the protein having mutations in an amino acid sequence of α-N-acetylgalactosaminidase and having α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
      (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
      (b) a protein containing an amino acid sequence in which one or several amino acids other than those located at the substituted sites are deleted, substituted or added in the amino acid sequence described in (a) above, the protein having α-galactosidase activity;
      (c) a protein containing an amino acid sequence represented by SEQ ID NO:6 in which the amino acid 202 is substituted by glutamic acid or aspartic acid while the amino acid 205 is substituted by leucine, valine, or isoleucine; and
      (d) a protein containing an amino acid sequence in which one or several amino acids other than those located at the substituted sites are deleted, substituted or added in the amino acid sequence described in (c) above, the protein having α-galactosidase activity,
   the active site specific chaperone being a compound represented by a general formula (I) below 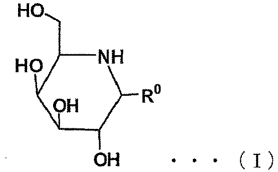 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
   the active site specific chaperone being a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.
<2> A stability improver for enhancing stability of a protein, in which
   the protein has mutations in an amino acid sequence of α-N-acetylgalactosaminidase and has α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
   (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
   (b) a protein containing an amino acid sequence in which one or several amino acids other than those located at the substituted sites are deleted, substituted or added in the amino acid sequence described in (a) above, the protein having α-galactosidase activity;
   (c) a protein containing an amino acid sequence represented by SEQ ID NO:6 in which the amino acid 202 is substituted by glutamic acid or aspartic acid while the amino acid 205 is substituted by leucine, valine, or isoleucine; and
   (d) a protein containing an amino acid sequence in which one or several amino acids other than those located at the substituted sites are deleted, substituted or added in the amino acid sequence described in (c) above, the protein having α-galactosidase activity,
   the stability improver including a compound selected from the group consisting of α-allo-homonojirimycin, α-galacto-homonojirimycin, α-1-C-butyl-deoxynojirimycin, calystegine A₃, calystegine B₂, N-methyl-calystegine A₃, N-methyl-calystegine B₂, and a compound represented by a general formula (I) below 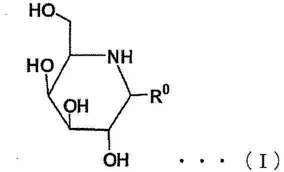 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group.
<3> A drug for treating Fabry disease, in which:
   the drug is used in combination with a protein which has mutations in an amino acid sequence of α-N-acetylgalactosaminidase and has α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
   (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
   (b) a protein containing an amino acid sequence in which one or several amino acids other than those located at the substituted sites are deleted, substituted or added in the amino acid sequence described in (a) above, the protein having α-galactosidase activity;
   (c) a protein containing an amino acid sequence represented by SEQ ID NO:6 in which the amino acid 202 is substituted by glutamic acid or aspartic acid while the amino acid 205 is substituted by leucine, valine, or isoleucine; and
   (d) a protein containing an amino acid sequence in which one or several amino acids other than those located at the substituted sites are deleted, substituted or added in the amino acid sequence described in (c) above, the protein having α-galactosidase activity,
   the drug including a compound selected from the group consisting of α-allo-homonojirimycin, α-galacto-homonojirimycin, α-1-C-butyl-deoxynojirimycin, calystegine A₃, calystegine B₂, N-methyl-calystegine A₃, N-methyl-calystegine B₂, and a compound represented by a general formula (I) below 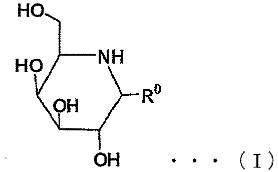 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group.
<4> A method for enhancing stability of a protein, the method including:
   a step of causing the protein to make contact with an active site specific chaperone,
   the protein having mutations in an amino acid sequence of α-N-acetylgalactosaminidase and having α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
      (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
      (b) a protein containing an amino acid sequence in which one or several amino acids other than those located at the substituted sites are deleted, substituted or added in the amino acid sequence described in (a) above, the protein having α-galactosidase activity;
      (c) a protein containing an amino acid sequence represented by SEQ ID NO:6 in which the amino acid 202 is substituted by glutamic acid or aspartic acid while the amino acid 205 is substituted by leucine, valine, or isoleucine; and
      (d) a protein containing an amino acid sequence in which one or several amino acids other than those located at the substituted sites are deleted, substituted or added in the amino acid sequence described in (c) above, the protein having α-galactosidase activity,
      the active site specific chaperone being a compound represented by a general formula (I) below 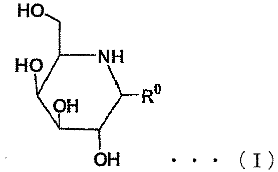 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
      the active site specific chaperone being a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.

### Advantageous Effects of Invention

The present invention provides (i) a pharmaceutical combination containing α-NAGA mutant for treatment of Fabry disease and (ii) use of the pharmaceutical combination. The present invention can be effectively used in treatment of Fabry disease.

### Brief Description of Drawings

Fig. 1 is a view illustrating inhibition of enzyme activity of an α-NAGA mutant by DGJ or GBS.
Fig. 2 is a view showing a Lineweaver-Burk plot of enzyme activity of an α-NAGA mutant obtained when DGJ or GBS is used and an inhibition constant Ki calculated from the plot.
Fig. 3 is a view showing a result of heat stability test for confirming inhibitory activity of DGJ with respect to an α-NAGA mutant.
Fig. 4 is a view showing results of measuring Tm values obtained when DGJ and GBS are used under an acidic condition and a neutral condition.
Fig. 5 is a view showing enzyme activity of an α-NAGA mutant which is measured when the α-NAGA mutant has been added simultaneously with various concentrations of DGJ to a cultivated fibroblast derived from a Fabry disease model mouse.

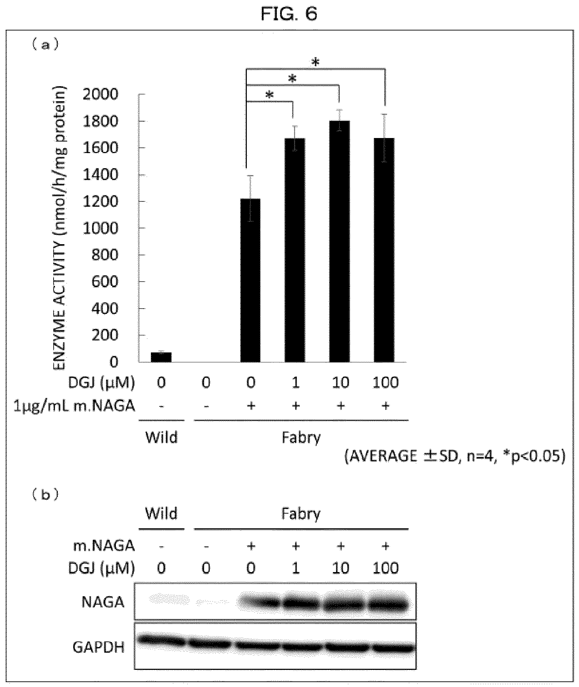

Fig. 6 is a view showing enzyme activity of an α-NAGA mutant and enzyme uptake amounts of the α-NAGA mutant which are measured when the α-NAGA mutant has been added simultaneously with various concentrations of DGJ to a cultivated fibroblast derived from a patient of Fabry disease. (a) of Fig. 6 shows enzyme activity of an α-NAGA mutant which is measured when the α-NAGA mutant has been added simultaneously with various concentrations of DGJ to a cultivated fibroblast derived from a patient of Fabry disease. (b) of Fig. 6 shows an enzyme uptake amount of the α-NAGA mutant which is measured when the α-NAGA mutant has been added simultaneously with various concentrations of DGJ to a cultivated fibroblast derived from a patient of Fabry disease.

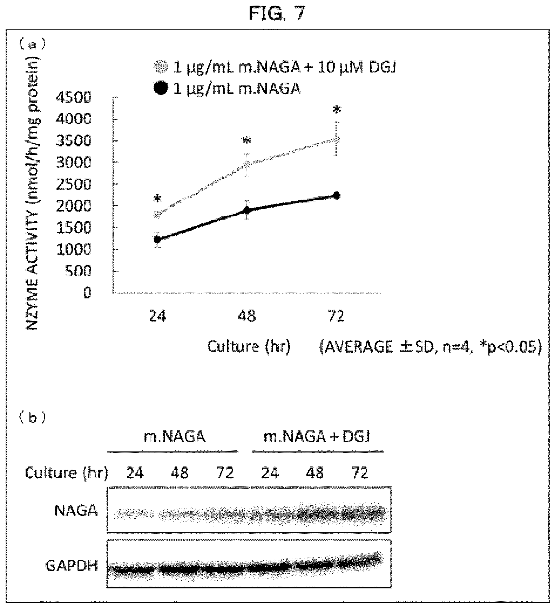

Fig. 7 is a view showing changes in enzyme activity and in enzyme uptake amount with time of an α-NAGA mutant which are measured when the α-NAGA mutant has been added simultaneously with DGJ to a cultivated fibroblast derived from a patient of Fabry disease. (a) of Fig. 7 shows a change in enzyme activity with time of an α-NAGA mutant which is measured when the α-NAGA mutant has been added simultaneously with DGJ to a cultivated fibroblast derived from a patient of Fabry disease, and (b) of Fig. 7 shows a change in enzyme uptake amount with time of the α-NAGA mutant which is measured when the α-NAGA mutant has been added simultaneously with DGJ to a cultivated fibroblast derived from a patient of Fabry disease.

### Description of Embodiments

### [Definition of terms]

Fabry disease is a glycolipid metabolic disorder which develops as follows: As a result of a decrease in the activity or deficiency of an enzyme called "α-galactosidase" present in a lysosome, one of the human intracellular organelles, a glycolipid called globotriaosylceramide (also referred to as ceramide trihexoside), which is an in vivo substrate of the enzyme, is not degraded and thus accumulated within the body (for example, the blood vessels, skins, cornea, nerves, kidneys, and heart).

Since a gene encoding α-galactosidase lies on the X chromosome, this disease has an X-chromosomal mode inheritance. Therefore, in this disease, a definite clinical feature is seen mainly in hemizygous males. It is believed that "classic Fabry disease", which takes a typical clinical course, develops in about one out of 40,000 male children. Symptoms such as pain in the hand and the foot (limb pain), hypohidrosis, angiokeratoma, and corneal opacity appear during the childhood and adolescence; these symptoms progress and then cause systemic organ damage such as renal failure, heart failure, and cerebrovascular disorder in middle age or later, which become a cause of death. There is also "late-onset Fabry disease", which does not take such a typical clinical course as "classic Fabry disease" and which develops late and takes a relatively moderate course. In patients with this type of disease, remaining α-galactosidase activity is observed though it is low. As a late-onset Fabry disease, for example, "cardiac Fabry disease" is known, which causes the above-mentioned glycolipid accumulation mainly in the heart. Consequently, cardiac hypertrophy occurs, and disorders such as heart failure and arrhythmia are caused. On the other hand, in heterozygous female Fabry disease patients, various types of clinical features are observed in accordance with the characteristics of the X chromosome. Specifically, cases range from serious cases which are similar to those of hemizygous males to cases in which substantially no symptoms are observed. However, according to recent research, it has become clear that most heterozygous female Fabry disease patients develop some sort of symptoms with age. There is a viewpoint that they should not be regarded as "carriers" but as "patients".

The term "enzyme replacement therapy" refers to introduction of a purified enzyme into an individual who is deficient in such an enzyme. An enzyme to be administered can be obtained from a natural source or by recombinant expression. This term also refers to introduction of a purified enzyme into an individual (e.g., an individual who suffers from protein deficiency) who requires administration of the purified enzyme with another method or benefits from such an administration of the purified enzyme. The enzyme to be introduced can be a purified recombinant enzyme produced in vitro or can be an enzyme that is purified from an isolated tissue or fluid (e.g., placenta or animal's milk) or from a plant.

When used herein, the terms "active site specific chaperone" and "pharmacological chaperone" refer to arbitrary molecules which cause reversible interaction specifically with an active site of protein to enhance formation of a stable molecular conformation. Those terms intend any molecules including proteins, peptides, nucleic acids, carbohydrates, and the like. Those terms do not mean chemical agents such as an endogenous chaperone (e.g., BiP) or a non-specific chemical chaperone (e.g., glycerol, DMSO, or deuterated water), which have been found to be chaperones that are not specific to various proteins.

When used herein, the term "active site" refers to a region of protein which region has some specific bioactivity. For example, the active site can be a site that binds to a substrate or another binding partner so as to contribute to an amino acid residue which is directly involved in preparation and destruction of a chemical bond. The active site of the present invention can encompass a catalytic site of enzyme, an antigen binding site of antibody, a ligand binding domain of receptor, a binding domain of controlling factor, and a receptor binding domain of secretory protein. The active site can also encompass DNA binding domains of a transactivation factor, a protein-protein interaction factor, a transcription factor, and a controlling factor.

In the present invention, the term "treatment" includes completely curing or relieving a symptom of the target disease, inhibiting symptom exacerbation of the target disease, and inhibiting or delaying the onset of the target disease. That is, the term "treatment" includes "prevention" where an individual has not developed the target disease.

### (Definition of terms in relation to α-NAGA mutant)

Herein, unless otherwise stated, the terms are defined as follows.

The terms "α-galactosidase" and "α-GAL" both refer to "human α-galactosidase A". The terms "α-N-acetylgalactosaminidase" and "α-NAGA" both refer to "human α-galactosidase B", i.e., "human α-N-acetylgalactosaminidase". The abbreviation "wt" stands for wild type. The abbreviation "M6P" stands for "mannose-6-phosphate". The term "α-GAL activity" refers to the later-described activity of hydrolyzing α-GAL substrate (see reaction formula (1) below), which is not limited to the activity of α-GAL protein (wild-type α-GAL). The term "α-NAGA activity" refers to the later-described activity of hydrolyzing α-NAGA substrate (see reaction formula (2) below), which is not limited to the activity of α-NAGA protein (wild-type α-NAGA). The phrase "acquired α-GAL activity" means that the binding reactivity with α-GAL substrate became relatively higher than that with α-NAGA substrate at the substrate-binding site. The phrase "have substrate specificity of α-GAL" means that the structure of an active site (particularly, position and type of an amino acid residue that plays an important role for the binding reactivity to the substrate) is the same as that of wild-type α-GAL.

The terms "amino acid 188" and "amino acid 191" refer to the positions of amino acids with respect to the amino acid sequence (SEQ ID NO:2) of wild-type α-NAGA (a position counted from the amino acid residue at the N-terminal (as amino acid 1) toward the C-terminal of this amino acid sequence).

The term "α-NAGA mutant" basically refers to any wild-type α-NAGA mutant, without being limited to a particular mutant (amino acid mutant). Herein, a mutant protein having serine at amino acid 188 substituted by glutamic acid, and alanine at amino acid 191 substituted by leucine in the amino acid sequence (SEQ ID NO:2) of wild-type α-NAGA (and thus referred to as "α-NAGA (S188E/A191L)") may be referred to (defined) as an α-NAGA mutant.

The term "α-GAL signal peptide-fused α-NAGA" generally refers to a protein having the signal peptide moiety of wild-type α-NAGA (a peptide moiety consisting of amino acids 1-17 of the amino acid sequence represented by SEQ ID NO:2) replaced with the signal peptide moiety of wild-type α-GAL (a peptide moiety consisting of amino acids 1-31 of the amino acid sequence represented by SEQ ID NO:10). Here, although the signal peptide moiety is generally removed upon extracellular secretion following the intracellular expression of the protein, according to the present invention, the protein after the extracellular secretion may also be referred to as "α-GAL signal peptide-fused α-NAGA" for the sake of convenience.

The terms "α-GAL signal peptide-fused α-NAGA mutant" and "α-GAL signal peptide-fused α-NAGA (S188E/A191L)" generally refer to a protein having the signal peptide moiety of an α-NAGA mutant (for example, a peptide moiety consisting of amino acids 1-17 of the amino acid sequence represented by SEQ ID NO:2) replaced with the signal peptide moiety of wild-type α-GAL (a peptide moiety consisting of amino acids 1-31 of the amino acid sequence represented by SEQ ID NO:10). Here, although the signal peptide moiety is generally removed upon extracellular secretion following the intracellular expression of the protein, according to the present invention, the protein after the extracellular secretion may also be referred to as "α-GAL signal peptide-fused α-NAGA mutant" and "α-GAL signal peptide-fused α-NAGA (S188E/A191L)" for the sake of convenience.

### (Description of sequence listing)

Enzyme proteins of the nucleotide sequences and amino acid sequences represented by SEQ ID NOS:1-10 in the present description are shown in Table A below. In Table A, the term "main body" refers to a part that becomes the mature protein without the signal peptide moiety. The symbol "+" represents binding between the indicated signal peptide and the main body.

### [Table 1]

**TABLE A**

| SEQ ID NO | Protein |
|---|---|
| SEQ ID NO: 1 (nucleotide sequence) | wt α-NAGA |
| SEQ ID NO: 2 (amino acid sequence) | (signal peptide of wt α-NAGA + main body of wt α-NAGA) |
| SEQ ID NO: 3 (nucleotide sequence) | α-NAGA mutant (α-NAGA (S188E/A191L)) |
| SEQ ID NO: 4 (amino acid sequence) | |
| | (signal peptide of wt α-NAGA + main body of α-NAGA mutant) |
| SEQ ID NO: 5 (nucleotide sequence) | signal peptide of wt α-GAL + main body of wt α-NAGA |
| SEQ ID NO: 6 (amino acid sequence) | |
| SEQ ID NO: 7 (nucleotide sequence) | signal peptide of wt α-GAL + main body of α-NAGA mutant wt α-GAL |
| SEQ ID NO: 8 (amino acid sequence) | |
| SEQ ID NO: 9 (nucleotide sequence) | |
| SEQ ID NO: 10 (amino acid sequence) | (signal peptide of wt α-GAL + main body of wt α-GAL) |

### [1. Method for enhancing stability of protein]

The present invention provides a method for enhancing stability of a protein, the method including a step of causing the protein to make contact with an active site specific chaperone, the protein having mutations in an amino acid sequence of α-N-acetylgalactosaminidase and having α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
(a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
(b) a protein containing an amino acid sequence in which one or several amino acids other than those located at the substituted sites are deleted, substituted or added in the amino acid sequence described in (a) above, the protein having α-galactosidase activity;
(c) a protein containing an amino acid sequence represented by SEQ ID NO:6 in which the amino acid 202 is substituted by glutamic acid or aspartic acid while the amino acid 205 is substituted by leucine, valine, or isoleucine; and
(d) a protein containing an amino acid sequence in which one or several amino acids other than those located at the substituted sites are deleted, substituted or added in the amino acid sequence described in (c) above, the protein having α-galactosidase activity,
   the active site specific chaperone being a compound represented by a general formula (I) below 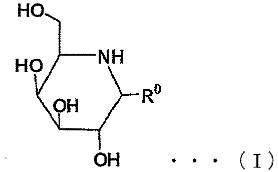 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
   the active site specific chaperone being a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.

The following description will first discuss details of proteins and active site specific chaperones which are applied to the method of the present invention.

The present inventors succeeded so far in creating an α-NAGA mutant as a novel and excellent highly functional enzyme which can be used to treat Fabry disease (see Patent Literatures 3 and 4).

The present invention is based on novel finding of a method for enhancing stability of the α-NAGA mutant.

### [Protein]

A protein of the present invention is specifically a mutant enzyme of α-N-acetylgalactosaminidase (α-NAGA).

Before now, the present inventors studied whether or not an enzyme other than α-GAL can be used as an enzyme used for enzyme replacement for treating Fabry disease. Specifically, the present inventors focused on "α-N-acetylgalactosaminidase (α-NAGA)", which is a lysosomal enzyme similar to α-GAL (meaning that the localization of α-NAGA in a cell is the same as that of α-GAL), and whose overall structure is very similar to that of α-GAL but with different substrate specificity.

α-NAGA is an enzyme which differs from α-GAL in a part of the structure of the substrate-binding site at the active site but is very similar to α-GAL with respect to the structure and properties regarding the other parts including the catalytic site. Therefore, the catalytic reaction mechanism of α-NAGA is very similar to the catalytic reaction mechanism of α-GAL with respect to, for example, the types of reaction substrate and reaction product.

Hence, the present inventors focused on α-NAGA as described above and found that when the substrate specificity of α-NAGA is modified to have α-galactosidase activity by altering the structure of the active site (in particular, the substrate-binding site) by gene manipulation of α-NAGA (for example, by substituting the α-NAGA-type amino acid residues that play the key role among the amino acid residues related to the substrate recognition of α-NAGA by the α-GAL-type amino acid residues), a novel excellent highly functional enzyme that can be used for treating Fabry disease can be created (see Patent Literatures 4 and 5).

More specifically, the protein of the present invention is a protein of (1a) or (1b) below, preferably a protein of (1c) below.
(1a) a protein which has acquired α-galactosidase (α-GAL) activity by altering the structure of the active site (in particular, the substrate-binding site) of wild-type α-NAGA, preferably a protein having the substrate specificity of α-GAL.
(1b) a protein of (1a) above with a signal peptide. Here, the type of the signal peptide is not particularly limited, and it may be a signal peptide derived from wild-type α-NAGA or other protein.
(1c) a protein of (1b) above, where the signal peptide is derived from wild-type α-GAL.

Herein, the phrase "acquired α-GAL activity" means, as already mentioned above, that the binding reactivity to a substrate of α-GAL becomes relatively higher than the binding reactivity to a substrate of α-NAGA at the substrate-binding site of α-NAGA. Accordingly, the above structural alteration is not limited to a structural alteration that binding between α-NAGA and the substrate thereof is completely impossible. Thus, the structural alteration may be one that makes the binding reactivity to the substrate of α-GAL significantly higher than the binding reactivity to the substrate of α-NAGA, which has originally been relatively significantly higher than the binding reactivity to the substrate of α-GAL. Furthermore, the phrase "having the substrate specificity of α-GAL", as already mentioned above, means that the structure of the active site (in particular, the positions and the types of amino acid residues which play an important role in the binding reactivity to a substrate) is the same as that of α-GAL.

In the present invention, the term "substrate of α-GAL" refers to a natural compound such as a glycolipid, e.g., globotriaosylceramide (ceramide trihexoside (CTH)), which has a galactose residue bound to the non-reducing end via α-bond, or a synthetic compound, e.g., 4-methylumbelliferyl-α-D-galactoside. The term "substrate of α-NAGA" refers to a natural compound such as an oligosaccharide, glycoprotein or glycolipid having an N-acetylgalactosamine residue bound to the non-reducing end via α-bond, or a synthetic compound, e.g., 4-methylumbelliferyl-α-N-acetyl-D-galactosaminide.

Here, a catalytic reaction of wild-type α-GAL is represented by reaction formula (1) below, and a catalytic reaction of wild-type α-NAGA is represented by reaction formula (2) below. 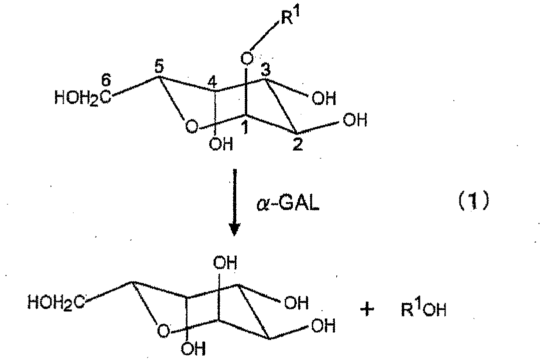 (In reaction formula (1), when the substrate is a natural compound, R¹ represents "a group derived from a sugar complex", and when the substrate is a synthetic compound, R¹ represents "a 4-methylumbelliferyl group".)

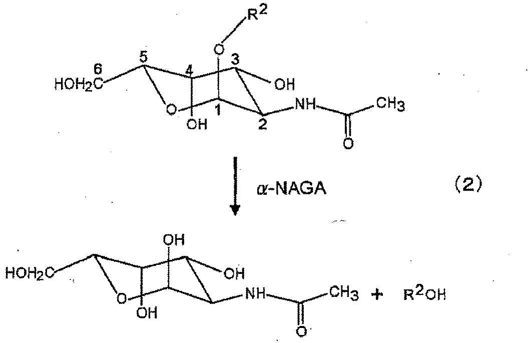

(In reaction formula (2), when the substrate is a natural compound, R² represents "a group derived from a sugar complex", and when the substrate is a synthetic compound, R² represents "a 4-methylumbelliferyl group".)

Preferable examples of the protein of the present invention include proteins comprising the amino acid sequence of the following (2a) or (2b), and having α-GAL activity.
(2a) an amino acid sequence in which at least one of the amino acids 188 and 191 included in the amino acid sequence of wild-type α-NAGA is substituted by another amino acid, preferably, an amino acid sequence in which both of the amino acids 188 and 191 are substituted by other amino acids.
(2b) an amino acid sequence in which one or several amino acids except the amino acids 188 and 191 included in the amino acid sequence of (2a) above are deleted, substituted or added.

Information on the amino acid sequence (SEQ ID NO:2) of the subunit of wild-type α-NAGA (homodimer) and information on the nucleotide sequence (SEQ ID NO:1) encoding the above amino acid sequence are published, for example, as "accession number: NM_000262" from GenBank, and registered in "entry name: NAGAB_HUMAN", accession number: P17050" with Swiss-Prot (available from http://tw.expasy.org/uniprot/). Similarly, information on the amino acid sequence (SEQ ID NO:10) of the subunit of wild-type α-GAL (homodimer) and information on the nucleotide sequence (SEQ ID NO:9) encoding the above amino acid sequence are published, for example, as "accession number: NP_000160" from GenBank, and registered in "entry name: AGAL_HUMAN", accession number: P06280" with Swiss-Prot (available from http://tw.expasy.org/uniprot/).

Herein, examples of the above "amino acid sequence in which one or several amino acids are deleted, substituted or added" preferably include amino acid sequences in which about one to ten amino acids, and preferably about one to five amino acids are deleted, substituted or added.

Furthermore, regarding "the protein composed of an amino acid sequence in which one or several amino acids are deleted, substituted or added", it is important that the protein can stably exhibit α-GAL activity. Therefore, for example, all or some (preferably, all) of the amino acids 28-31, the amino acids 77-81, the amino acids 117-127, the amino acids 150-158, the amino acid 192, the amino acids 209-220 and the amino acids 242-254, which are believed to be important for the binding performance (substrate-binding performance) with an α-galactose residue of a substrate of α-GAL and the catalytic reactivity to the substrate (in particular, the 156th and 217th aspartic acids (Asp: D) at the catalytic site; the 45th aspartic acid (Asp: D) and the 350th arginine (Arg: R) that are believed to be important for forming a homodimer; and the amino acids 124, 177, 201, 359 and 385 (all of which being asparagine (Asn: N)) as N-type-sugar-chain-binding sites) are preferably not mutated (deleted, substituted or added) from the amino acid sequence of wild-type α-NAGA.

The other amino acid residue substituting for the amino acid residue 188 is not particularly limited as long as the amino acid residue is not serine (Ser: S). For example, glutamic acid (Glu: E) and aspartic acid (Asp: D) are preferable, and glutamic acid is more preferable. Similarly, the other amino acid residue substituting for the amino acid residue 191 is not particularly limited as long as the amino acid residue is not alanine (Ala: A). For example, leucine (Leu: L), valine (Val: V), isoleucine (Ile: I), phenylalanine (Phe: F) and methionine (Me: M) are preferable, and leucine is more preferable. Particularly preferable amino acids for substitution among them are glutamic acid for the amino acid 188 and leucine for the amino acid 191. Note that, preferably, the amino acids after the substitution do not substantially affect the structure composed of the rest of the unsubstituted amino acids. From this point of view, in a particularly preferable substitution embodiment, the amino acid residue 188 is glutamic acid and the amino acid residue 191 is leucine.

By substituting the amino acid 188 and the amino acid 191 present at the substrate-binding sites as described above, the following effects can be achieved. Specifically, regarding the amino acid residue 188, the interaction with the N-acetyl group (in particular, the oxygen atom) in the substrate of α-NAGA can be canceled, while producing a binding action with the hydroxyl group in the substrate of α-GAL. Regarding the amino acid residue 191, the interaction with the N-acetyl group (in particular, the methyl group) in the substrate of α-NAGA can be canceled, while limiting the binding space of the substrate (in particular, the space into which the N-acetyl group is incorporated). As a result, the recombinant enzyme (recombinant protein) obtained after the substitution of the amino acids has a binding reactivity to the substrate of α-GAL higher than the binding reactivity to the substrate of α-NAGA, and thus can serve as an enzyme with α-GAL activity significantly higher than α-NAGA activity. The recombinant enzyme having an amino acid sequence in which at least the amino acid 188 (serine) is substituted by glutamic acid and the amino acid 191 (alanine) is substituted by leucine is particularly preferable from the standpoint that the above-described effects can satisfactorily be achieved.

In addition, the protein of the present invention is preferably a protein described in (3a) or (3b) below:
(3a) a protein containing any one of the amino acid sequences described in (i) to (iii) below:
   (i) an amino acid sequence composed of the amino acids 18-411 of the amino acid sequence represented by SEQ ID NO:2 in which the amino acid 188 is substituted by an amino acid other than serine;
   (ii) an amino acid sequence composed of the amino acids 18-411 of the amino acid sequence represented by SEQ ID NO:2 in which the amino acid 191 is substituted by an amino acid other than alanine; or
   (iii) an amino acid sequence composed of the amino acids 18-411 of the amino acid sequence represented by SEQ ID NO:2 in which the amino acid 188 is substituted by an amino acid other than serine while the amino acid 191 is substituted by an amino acid other than alanine; or
(3b) a protein containing any one of the amino acid sequences described in (i) to (iii) of (3a) above in which one or several amino acids other than the amino acid(s) at the substituted site(s) are deleted, substituted or added, and having α-galactosidase activity.
   The amino acid sequence represented by SEQ ID NO:2 is an amino acid sequence composed of 411 amino acids constituting wild-type α-NAGA.

Specifically, the protein described in (3a) above is a protein composed of the amino acid sequence represented by SEQ ID NO:2 substituted as described in (i) to (iii), where it consists of an amino acid sequence ranging from the amino acids 18 to 411 and excluding the amino acids 1-17 that constitute the signal peptide of wild-type α-NAGA. As described above, the amino acid residues 188 and 191 are each one of the amino acids constituting the substrate-binding sites.

Here, a preferable example of the amino acid sequence containing an amino acid sequence ranging from the amino acids 18 to 411 may be an amino acid sequence having a signal peptide linked to the N-terminal of the amino acid sequence ranging from the amino acids 18 to 411. The signal peptide is not limited as long as it allows the protein to pass through a cell membrane of an affected organ. For example, a signal peptide is preferably a signal peptide of a lysosomal enzyme such as wild-type α-NAGA or wild-type α-GAL or a signal peptide of a secretase such as preprotrypsin, more preferably a signal peptide of wild-type α-NAGA or wild-type α-GAL, and still more preferably a signal peptide of wild-type α-GAL. The signal peptide of wild-type α-NAGA, as already described above, is a peptide composed of the amino acids 1-17 of the amino acid sequence of wild-type α-NAGA represented by SEQ ID NO:2. The signal peptide of wild-type α-GAL is a peptide composed of the amino acids 1-31 included in the amino acid sequence of wild-type α-GAL represented by SEQ ID NO:10. The signal peptide of preprotrypsin is a peptide composed of the amino acid sequence represented by SEQ ID NO:12. As the protein described in (3a), a protein containing the amino acid sequence described in (iii) is particularly preferable among proteins containing the amino acid sequences described in (i), (ii) and (iii).

A preferable example of the protein described in (3a) above includes a protein in which "the amino acid other than serine" described in (i) and (iii) above is glutamic acid or aspartic acid. Similarly, another preferable example of the protein described in (3a) above includes a protein in which "the amino acid other than alanine" described in (ii) and (iii) is one selected from the group consisting of leucine, valine, isoleucine, phenylalanine and methionine.

Furthermore, a particularly preferable example of the protein described in (3a) above includes a protein in which "the amino acid other than serine" described in (i) to (iii) is glutamic acid and "the amino acid other than alanine" described in (i) to (iii) is leucine. A preferable example of such protein includes a protein (α-NAGA (S188E/A191L)) in which the 188th serine is substituted by glutamic acid and the 191st alanine is substituted by leucine in the amino acid sequence of wild-type α-NAGA (SEQ ID NO:2) (see SEQ ID NO:4). In general, the alphabetical notation of an amino acid is expressed in three letters (e.g., "Ser") or one letter (e.g., "S"). The alphabetical letter preceding the number (e.g., "188") representing the position of an amino acid counted from the N-terminal represents an amino acid in one-letter notation before the substitution, while the alphabetical letter following the number represents an amino acid in one-letter notation after the substitution. Accordingly, for example, the notation "S188E" represents that the 188th Ser is substituted by Glu (the same similarly applies hereinafter).

The protein described in (3b) above is not limited as long as the protein contains an amino acid sequence in which one or several (for example, about one to ten, and preferably about one to five) amino acids other than the amino acid(s) located at the substituted site(s) are deleted, substituted or added in any one of the amino acid sequences described in (i) to (iii) above included in the protein described in (3a) above, and has α-GAL activity. Herein, the term "the substituted site" refers to, among the 394 amino acid residues constituting the amino acid sequences described in (i) to (iii), the amino acid residue corresponding to the position of the amino acid 188 in the amino acid sequence represented by SEQ ID NO:2 (provided that the amino acid sequence is limited to the amino acid sequence described in (i) or (iii)), and the amino acid residue corresponding to the position of the amino acid 191 in the amino acid sequence represented by SEQ ID NO:2 (provided that the amino acid sequence is limited to the amino acid sequence described in (ii) or (iii)). More specifically, the former amino acid residue refers to the amino acid residue 171 in the amino acid sequence described in (i) or (iii), and the latter amino acid residue refers to the amino acid residue 174 in the amino acid sequence described in (ii) or (iii).

Note that it is important that the protein described in (3b) above is a protein that can stably exhibit α-GAL activity. Therefore, for example, amino acid residues which are believed to be important for binding performance (substrate-binding performance) with an α-galactose residue of a substrate of α-GAL and the catalytic reactivity to the substrate are preferably amino acid residues that are not mutated (deleted, substituted or added) from the amino acid sequences described in (i) to (iii) above. Preferable examples of such amino acid residues include, among the amino acid residues constituting the amino acid sequences described in (i) to (iii) above, amino acid residues corresponding to the positions of the amino acids 28-31, the amino acids 77-81, the amino acids 117-127, the amino acids 150-158, the amino acid 192, the amino acids 209-220, and the amino acids 242-254 (in particular, the aspartic acids 156 and 217 (Asp: D) at the catalytic site) in the amino acid sequence represented by SEQ ID NO:2.

Similarly, amino acid residues which are believed to be important for forming a homodimer are also preferably amino acid residues that are not mutated (deleted, substituted or added) from the amino acid sequences described in (i) to (iii) above. Preferable examples of such amino acid residues include, among the amino acid residues constituting the amino acid sequences described in (i) to (iii) above, amino acid residues corresponding to the positions of the amino acids 45 and 350 (specifically, the 45th aspartic acid (Asp: D) and the 350th arginine (Arg: R)) in the amino acid sequence represented by SEQ ID NO:2.

Furthermore, amino acid residues which are N-type-sugar-chain-binding sites are also preferably not mutated (deleted, substituted or added) from the amino acid sequences described in (i) to (iii) above. Preferable examples of such amino acid residues include, among the amino acid residues constituting the amino acid sequences described in (i) to (iii) above, amino acid residues corresponding to the positions of the amino acids 124, 177, 201, 359 and 385 (all of which being asparagine (Asn: N)) in the amino acid sequence represented by SEQ ID NO:2. In addition, a protein of the invention is preferably a protein of (4a) or (4b) below.
(4a) a protein containing any one of the amino acid sequences described in (i) to (iii) below:
   (i) an amino acid sequence having the amino acid 202 substituted by an amino acid other than serine in the amino acid sequence represented by SEQ ID NO:6;
   (ii) an amino acid sequence having the amino acid 205 substituted by an amino acid other than alanine in the amino acid sequence represented by SEQ ID NO:6; or
   (iii) an amino acid sequence having the amino acid 202 substituted by an amino acid other than serine and the amino acid 205 substituted by an amino acid other than alanine in the amino acid sequence represented by SEQ ID NO:6; or
(4b) a protein containing an amino acid sequence in which one or several amino acids other than those located at the substituted site(s) are deleted, substituted or added in any one of the amino acid sequences described in (i) to (iii) above, the protein having α-galactosidase activity.

The amino acid sequence represented by SEQ ID NO:6 is an amino acid sequence (with a total of 425 amino acids) in which the amino acids 1-17 corresponding to the signal peptide moiety are altered by the signal peptide moiety of wild-type α-GAL in an amino acid sequence composed of 411 amino acids constituting wild-type α-NAGA, which is a so-called fusion protein. Here, the signal peptide moiety of the wild-type α-GAL, as already described above, is a peptide moiety of the amino acids 1-31 among the amino acid sequence constituting the wild-type α-GAL represented by SEQ ID NO:10.

In the amino acid sequence constituting the protein of (4a) above, the amino acid residues 188 and 191 are each one of the amino acids constituting the substrate-binding sites. As the protein described in (4a), a protein containing the amino acid sequence described in (iii) is particularly preferable among proteins containing the amino acid sequences described in (i), (ii) and (iii).

A preferable example of the protein described in (4a) above includes a protein in which "the amino acid other than serine" described in (i) and (iii) above is glutamic acid or aspartic acid. Similarly, another preferable example of the protein described in (4a) above includes a protein in which "the amino acid other than alanine" described in (ii) and (iii) is one selected from the group consisting of leucine, valine, isoleucine, phenylalanine and methionine.

Furthermore, a particularly preferable example of the protein described in (4a) above includes a protein in which "the amino acid other than serine" described in (i) to (iii) is glutamic acid and "the amino acid other than alanine" described in (i) to (iii) is leucine. A preferable example of such protein includes a protein (α-GAL signal peptide-fused α-NAGA (S202E/A205L)) having the 202nd serine substituted by glutamic acid and the 205th alanine substituted by leucine in the amino acid sequence represented by SEQ ID NO:6.

The protein described in (4b) above is not limited as long as the protein contains an amino acid sequence in which one or several (for example, about one to ten, and preferably about one to five) amino acids other than the amino acid(s) located at the substituted site(s) are deleted, substituted or added in any one of the amino acid sequences described in (i) to (iii) above included in the protein described in (4a) above, and has α-GAL activity. Herein, the term "the substituted site" refers to the amino acid residue 202 (provided that the amino acid sequence is limited to the amino acid sequence described in (i) or (iii)) and the amino acid residue 205 (provided that the amino acid sequence is limited to the amino acid sequence described in (ii) or (iii)) among the 425 amino acid residues constituting the amino acid sequences described in (i) to (iii) above.

Note that it is important that the protein described in (4b) above is a protein that can stably exhibit α-GAL activity. Therefore, for example, amino acid residues which are believed to be important for binding performance (substrate-binding performance) with an α-galactose residue of a substrate of α-GAL and the catalytic reactivity to the substrate are preferably amino acid residues that are not mutated (deleted, substituted or added) from the amino acid sequences described in (i) to (iii) above. Preferable examples of such amino acid residues include amino acid residues 42-45, 91-95, 131-141, 164-172, 206, 223-234 and 256-268 (in particular, the 170th and 231st aspartic acids (Asp: D) at the catalytic site) among the 425 amino acid residues constituting the amino acid sequences described in (i) to (iii) above.

Similarly, amino acid residues which are believed to be important for forming a homodimer are also preferably amino acid residues that are not mutated (deleted, substituted or added) from the amino acid sequences described in (i) to (iii) above. Preferable examples of such amino acid residues include amino acid residues 59 and 364 (specifically, the 59th aspartic acid (Asp: D) and the 364th arginine (Arg: R)) among the 425 amino acid residues constituting the amino acid sequences described in (i) to (iii) above.

Furthermore, amino acid residues which are N-type-sugar-chain-binding sites are also preferably not mutated (deleted, substituted or added) from the amino acid sequences described in (i) to (iii) above. Preferable examples of such amino acid residues include amino acid residues 138, 191, 215, 373 and 399 (all of which being asparagine (Asn: N)) among the 425 amino acid residues constituting the amino acid sequences described in (i) to (iii) above.

With respect to the above-described proteins of the present invention, α-GAL activity can be measured as follows. For example, a target protein is expressed in a cell derived from a mammal, such as a CHO cell or a human fibroblast, and collected therefrom. The protein (enzyme solution) is then mixed with 4-methylumbelliferyl-α-D-galactoside (a synthetic substrate obtained from α-D-galactose and 4-methylumbelliferone (fluorogenic substrate)), and the mixture is allowed to react under an acidic condition. The amount of 4-methylumbelliferone released is detected as a unit quantity of the enzyme solution per unit time to measure the α-GAL activity.

α-NAGA activity can also be measured as follows. A target protein is expressed and collected in a similar manner to the measurement for the α-GAL activity above. The protein (enzyme solution) is then mixed with 4-methylumbelliferyl-α-N-acetyl-D-galactosaminide (a synthetic substrate obtained from α-N-acetyl-D-galactosamine and 4-methylumbelliferone (fluorogenic substrate)), and the mixture is allowed to react under an acidic condition. The amount of 4-methylumbelliferone which can be released is detected as a unit quantity of the enzyme solution per unit time to measure the α-NAGA activity.

In the above methods of measuring α-GAL activity and α-NAGA activity, various types of known detection methods can be employed for detecting the fluorogenic substrate. For example, a detection method using a fluorophotometer or the like is preferable. The target protein can be expressed by incorporating a gene encoding the protein into a known expression vector or the like, and then introducing the vector into a cell.

### (Recombinant gene)

The above-described protein of the present invention is encoded by a recombinant gene.

A gene of this section codes for the above-described protein of the present invention. Preferable examples of the gene that codes for the protein of the present invention include genes including DNA described in (1a) or (1b) below. Although the DNAs described in (1a) and (1b) are preferably structural genes of the protein of the present invention, the gene including any of these DNAs is not limited thereto and it may consist of the DNA alone or it may contain the DNA together with other known nucleotide sequences required for gene expression (such as a transcriptional promoter, SD sequence, Kozak sequence and a terminator).
(1a) DNA containing any one of the nucleotide sequences described in (i) to (iii) below;
   (i) a nucleotide sequence of the nucleotides 52-1236 of the nucleotide sequence represented by SEQ ID NO:1 in which the nucleotides 562-564, "agc", are substituted by nucleotides representing a codon of an amino acid other than serine; (ii) a nucleotide sequence of the nucleotides 52-1236 of the nucleotide sequence represented by SEQ ID NO:1 in which the nucleotides 571-573, "gcc", are substituted by nucleotides representing a codon of an amino acid other than alanine; or (iii) a nucleotide sequence of the nucleotides 52-1236 of the nucleotide sequence represented by SEQ ID NO:1 in which the nucleotides 562-564, "agc", are substituted by nucleotides representing a codon of an amino acid other than serine while the nucleotides 571-573 are substituted by nucleotides representing a codon of an amino acid other than alanine; or
(1b) DNA which encodes a protein having α-galactosidase activity and which hybridizes with DNA composed of a nucleotide sequence complementary to DNA containing any one of the nucleotide sequences described in (i) to (iii) of (1a) above under stringent conditions, wherein nucleotides are the same as the nucleotides at the substituted site above at the corresponding position.

In the present invention, the term "codon" refers not to only the triple base linkage (triplet) of an RNA sequence after transcription but also the triple base linkage of a DNA sequence. Accordingly, codons in the case of a DNA sequence are denoted using thymine (T) instead of uracil (U).

The nucleotide sequence represented by SEQ ID NO:1 is a nucleotide sequence composed of 1,236 nucleotides encoding wild-type α-NAGA.

More specifically, the DNA described in (1a) above is DNA composed of a nucleotide sequence containing the nucleotides 52-1236 of the nucleotide sequence represented by SEQ ID NO:1 and excluding the nucleotides 1-51 that encodes a signal peptide of wild-type α-NAGA, in which nucleotides are replaced as described in (i) to (iii).

Here, a preferable example of the nucleotide sequence containing the nucleotides 52-1236 includes a nucleotide sequence in which a nucleotide sequence (polynucleotide) encoding a signal peptide is linked to the 5' side of the nucleotides 52-1236. The signal peptide is not limited as long as it allows the protein to pass through a cell membrane of an affected organ. For example, the signal peptide is preferably a signal peptide of a lysosomal enzyme such as wild-type α-NAGA or wild-type α-GAL, or a signal peptide of a secretase such as preprotrypsin, more preferably a signal peptide of wild-type α-NAGA or wild-type α-GAL, and still more preferably a signal peptide of wild-type α-GAL. A nucleotide sequence encoding the signal peptide of wild-type α-NAGA is a nucleotide sequence composed of the nucleotides 1-51 of the nucleotide sequence of wild-type α-NAGA represented by SEQ ID NO:1. A nucleotide sequence encoding the signal peptide of wild-type α-GAL is a nucleotide sequence composed of the nucleotides 1-93 of the nucleotide sequence of wild-type α-GAL represented by SEQ ID NO:9. A nucleotide sequence encoding the signal peptide of preprotrypsin is a nucleotide sequence represented by SEQ ID NO:11.

As the DNA described in (1a) above, DNA containing the nucleotide sequence described in (iii) is particularly preferable among the DNAs containing the nucleotide sequence described in (i), (ii) or (iii). In addition, the DNA described in (1a) above is preferably DNA in which "the nucleotides representing a codon of an amino acid other than serine" described in (i) and (iii) are nucleotides representing a codon of glutamic acid or aspartic acid. Similarly, the DNA described in (1a) above is preferably DNA in which "the nucleotides representing a codon of an amino acid other than alanine" described in (ii) and (iii) are nucleotides representing a codon of one selected from the group consisting of leucine, valine, isoleucine, phenylalanine and methionine. Herein, regarding nucleotides representing a codon of each of the above amino acids (wherein the nucleotide at the left end is defined as the nucleotide at the 5' side), nucleotides representing a codon of glutamic acid are "gag" or "gaa" (preferably "gag"), and nucleotides representing a codon of aspartic acid are "gat" or "gac". Similarly, nucleotides representing a codon of leucine are "ctc", "ctt", "cta" or "ctg" (preferably "ctc"), nucleotides representing a codon of valine are "gtt", "gtc", "gta" or "gtg", nucleotides representing a codon of isoleucine are "att", "atc" or "ata", nucleotides representing a codon of phenylalanine are "ttt" or "ttc", and nucleotides representing a codon of methionine are "atg". Nucleotides representing a codon of serine include "agt" in addition to "agc" mentioned above, and nucleotides representing a codon of alanine include "gct", "gca" and "gcg" in addition to "gcc" mentioned above.

Furthermore, a particularly preferable DNA described in (1a) above is DNA in which "the nucleotides representing a codon of an amino acid other than serine" described in (i) to (iii) are nucleotides representing a codon of glutamic acid, and "the nucleotides representing a codon of an amino acid other than alanine" are nucleotides representing a codon of leucine. A preferable example of such DNA includes DNA composed of a nucleotide sequence (SEQ ID NO:3) in which the nucleotides 562-564 representing a codon of serine included in the nucleotide sequence of wild-type α-NAGA (SEQ ID NO:1) are substituted by nucleotides representing a codon of glutamic acid ("agc"→"gag"), and the nucleotides 571-573 representing a codon of alanine are substituted by nucleotides representing a codon of leucine ("gcc"→"ctc"). In this exemplification, the nucleotides 562-564 after the substitution may be nucleotides other than "gag" as long as they represent a codon of glutamic acid. Similarly, the nucleotides 571-573 after the substitution may be nucleotides other than "ctc" mentioned above as long as they represent a codon of leucine.

Such substituted-type DNA mutant can be prepared in accordance with a site-directed mutagenesis method described in, for example, Molecular Cloning, A Laboratory Manual 2nd ed., Cold Spring Harbor Laboratory Press (1989), Current Protocols in Molecular Biology, John Wiley & Sons (1987-1997). Specifically, such DNA can be prepared by a known method such as a Kunkel method or a Gapped duplex method using a kit for introducing a mutation utilizing the site-directed mutagenesis method. Preferable examples of the kit include QuickChange™ Site-Directed Mutagenesis Kit (manufactured by Stratagene), GeneTailor™ Site-Directed Mutagenesis System (manufactured by Invitrogen Corporation), and TaKaRa Site-Directed Mutagenesis System (e.g., Mutan-K or Mutan-Super Express Km: manufactured by Takara Bio Inc.).

Alternatively, such DNA can be prepared by performing PCR under appropriate conditions using PCR primers designed such that a missense mutation is introduced to give nucleotides representing a codon of a desired amino acid, and using, as a template, for example, DNA containing a nucleotide sequence encoding wild-type α-NAGA. A DNA polymerase used for the PCR is not limited, but a DNA polymerase with high accuracy is preferable. Preferable examples thereof include Pwo DNA Polymerase (Roche Diagnostics K.K.), Pfu DNA polymerase (Promega), platinum Pfx DNA polymerase (Invitrogen Corporation), KOD DNA polymerase (Toyobo Co., Ltd.) and KOD-plus-polymerase (Toyobo Co., Ltd.). Reaction conditions for the PCR can appropriately be determined in accordance with, for example, the optimum temperature of the DNA polymerase used, and the length and the type of DNA to be synthesized. For example, preferable conditions include a total of 20 to 200 cycles of "5 to 30 seconds at 90°C to 98°C (thermal denaturation and dissociation)→5 to 30 seconds at 50°C to 65°C (annealing)→30 to 1200 seconds at 65°C to 80°C (synthesis and elongation)".

The DNA described in (1b) above can be obtained from a cDNA library or a genomic library by a known hybridization method such as colony hybridization, plaque hybridization or Southern blotting by using DNA containing any one of the nucleotide sequences described in any one of (i) to (iii) (i.e., DNA described in (1a)), DNA composed of a nucleotide sequence complementary to this DNA, or a fragment thereof as a probe. A library may be prepared by a known method, or a commercially available cDNA library or genomic library may be used. The library is not limited thereto.

As to a detailed procedure of the hybridization method, refer to, for example, Molecular Cloning, A Laboratory Manual 2nd ed. (Cold Spring Harbor Laboratory Press (1989)) as needed.

The term "stringent conditions" used upon performing a hybridization method refers to conditions during washing after hybridization, and specifically, a salt concentration of a buffer in the range of 15 to 330 mM and a temperature in the range of 25°C to 65°C, and preferably, a salt concentration in the range of 15 to 150 mM and a temperature in the range of 45°C to 55°C. More specifically, an example of the stringent conditions includes 80 mM at 50°C. In addition to the salt concentration, the temperature and the like, in consideration of other conditions such as the probe concentration, the length of the probe and the reaction time may also be considered to appropriately determine the conditions for obtaining the DNA described in (1b).

The DNA to be hybridized is a nucleotide sequence having a homology of preferably at least 40% or more, more preferably 60% or more, still more preferably 90% or more, particularly preferably 95% or more, and most preferably 99% or more relative to the nucleotide sequence of the DNA described in (1a) above.

Furthermore, in the DNA described in (1b) above, nucleotides are the same as the nucleotides at the substituted site above at the corresponding position.

Herein, the term "substituted site" refers to a site of the nucleotide substitution performed in the nucleotide sequences described in any one of (i) to (iii) contained in the DNA described in (1a) above. Specifically, the term refers to a site of nucleotides (triplet) representing a substituting codon caused by the nucleotide substitution. More specifically, the term "substituted site" refers to nucleotides corresponding to the positions of the nucleotides 562-564 of the nucleotide sequence represented by SEQ ID NO:1 (provided that the nucleotide sequences are limited to the nucleotide sequences described in (i) and (iii)), and nucleotides corresponding to the positions of the nucleotides 571-573 of the nucleotide sequence represented by SEQ ID NO:1 (provided that the nucleotide sequences are limited to the nucleotide sequences described in (ii) and (iii)) among the 1185 nucleotides constituting the nucleotide sequences described in (i) to (iii). More specifically, the former nucleotides refer to the nucleotides 511-513 of the nucleotide sequences described in (i) and (iii) above, and the latter nucleotides refer to the nucleotides 520-522 of the nucleotide sequences described in (ii) and (iii) above.

In addition, the "nucleotides" in the phrase "nucleotides corresponding to the nucleotides at the substituted site" refer to nucleotides (triplet) which are positioned to oppose nucleotides (triplet) complementary to the nucleotides at the substituted site in a hybrid prepared by hybridizing the DNA described in (1b) above with a strand complementary to the DNA described in (1a) above. For example, when the nucleotide sequence of the DNA described in (1b) above does not have a mutation such as deletion or addition, as compared with the DNA described in (1a) above (that is, when the lengths (the numbers of nucleotides) of both DNAs are the same), the nucleotides 511-513 and/or the nucleotides 520-522 of the nucleotide sequence of the DNA described in (1b) are the "nucleotides" in the phrase "nucleotides corresponding to the nucleotides at the substituted site".

It is important that the DNA described in (1b) above is DNA that encodes a protein having α-GAL activity. Therefore, for example, nucleotides representing a codon of an amino acid residue which is believed to be important for the binding performance (substrate-binding performance) with an α-galactose residue of a substrate of α-GAL and the catalytic reactivity to the substrate are preferably nucleotides that are not mutated (deleted, substituted or added) from the nucleotide sequences described in (i) to (iii) above. Preferable examples of such nucleotides of the nucleotide sequences described in (i) to (iii) include nucleotides corresponding to the positions of the nucleotides 82-93 (4 codons), 229-243 (5 codons), 349-381 (11 codons), 448-474 (9 codons), 574-576 (1 codon), 625-660 (12 codons) and 724-762 (13 codons) of the nucleotide sequence represented by SEQ ID NO:1 among the nucleotide sequences described in (i) to (iii). Among these nucleotides, nucleotides corresponding to the nucleotides 466-468 and 649-651, which represent codons of amino acid residues at catalytic sites, are particularly preferable.

Furthermore, in the DNA described in (1b) above, nucleotides representing a codon of an amino acid residue which is believed to be important for forming a homodimer are also preferably nucleotides that are not mutated (deleted, substituted or added) from the nucleotide sequences described in (i) to (iii). Preferable examples of such nucleotides of the nucleotide sequences described in (i) to (iii) include nucleotides corresponding to the positions of the nucleotides 133-135 and 1,048-1,050 of the nucleotide sequence represented by SEQ ID NO:1 among the nucleotide sequences described in (i) to (iii) above.

Furthermore, in the DNA described in (1b) above, nucleotides representing a codon of an amino acid residue as an N-type-sugar-chain-binding site are also preferably nucleotides that are not mutated (deleted, substituted or added) from the nucleotide sequences described in (i) to (iii) above. Preferable examples of such nucleotides of the nucleotide sequences described in (i) to (iii) above include nucleotides corresponding to the positions of the nucleotides 370-372, 529-531, 601-603, 1,075-1,077 and 1,153-1,155 of the nucleotide sequence represented by SEQ ID NO:1 among the nucleotide sequences described in (i) to (iii).

A particularly preferable example of the DNA described in (1b) above is DNA composed of a nucleotide sequence which is not completely identical to the nucleotide sequence of the DNA described in (1a) above but which is completely identical to the amino acid sequence after translation (i.e., DNA obtained by performing a silent mutation on the DNA described in (1a) above).

Preferable examples of a gene of the invention also include genes including DNA described in (2a) or (2b) below. Although the DNAs described in (2a) and (2b) are both preferably structural genes of the protein of the present invention, the gene including either of these DNAs is not limited thereto and it may consist of the DNA alone or it may contain the DNA together with other known nucleotide sequences required for gene expression (such as a transcriptional promoter, SD sequence, Kozak sequence and a terminator).
(2a) DNA containing any one of the nucleotide sequences described in (i) to (iii) below;
   (i) a nucleotide sequence having the nucleotides 604-606, "agc", of the nucleotide sequence represented by SEQ ID NO:5 substituted by nucleotides representing a codon of an amino acid other than serine;
   (ii) a nucleotide sequence having the nucleotides 613-615, "gcc", of the nucleotide sequence represented by SEQ ID NO:5 substituted by nucleotides representing a codon of an amino acid other than alanine; and
   (iii) a nucleotide sequence having the nucleotides 604-606, "agc", of the nucleotide sequence represented by SEQ ID NO:5 substituted by nucleotides representing a codon of an amino acid other than serine, and the nucleotides 613-615, substituted by nucleotides representing a codon of an amino acid other than alanine; or
(2b) DNA which encodes a protein having α-galactosidase activity and which hybridizes with DNA composed of a nucleotide sequence complementary to DNA containing a nucleotide sequences described in any one of (i) to (iii) of (2a) above under stringent conditions, wherein nucleotides are the same as the nucleotides at the substituted site above at the corresponding position.

The nucleotide sequence represented by SEQ ID NO:5 is a nucleotide sequence having the nucleotides 1-51 coding for a signal peptide moiety of a nucleotide sequence composed of 1,236 nucleotides encoding wild-type α-NAGA altered by a nucleotide sequence coding for a signal peptide moiety of wild-type α-GAL, namely, a nucleotide sequence coding for a so-called fusion protein. Here, the nucleotide sequence coding for the signal peptide moiety of the wild-type α-GAL, as already described above, is a sequence of nucleotides 1-93 of the nucleotide sequence coding for wild-type α-GAL represented by SEQ ID NO:9.

As the DNA described in (2a), DNA containing the nucleotide sequence described in (iii) is particularly preferable among the DNAs containing the nucleotide sequence described in (i), (ii) or (iii). In addition, a preferable example of the DNA described in (2a) above includes DNA in which "the nucleotides representing a codon of an amino acid other than serine" described in (i) and (iii) are nucleotides representing a codon of glutamic acid or aspartic acid. Similarly, a preferable example of the DNA described in (2a) above also includes DNA in which "the nucleotides representing a codon of an amino acid other than alanine" described in (ii) and (iii) are nucleotides representing a codon of one selected from the group consisting of leucine, valine, isoleucine, phenylalanine and methionine. Herein, the explanation for the DNA described in (1a) above similarly applies to the nucleotides representing a codon of each of the above amino acids.

Furthermore, a particularly preferable example of the DNA described in (2a) above includes DNA in which "the nucleotides representing a codon of an amino acid other than serine" described in (i) to (iii) are nucleotides representing a codon of glutamic acid, and "the nucleotides representing a codon of an amino acid other than alanine" are nucleotides representing a codon of leucine. A preferable example of such DNA is DNA composed of a nucleotide sequence (SEQ ID NO:7) in which the nucleotides 604-606 representing a codon of serine are substituted by nucleotides representing a codon of glutamic acid ("agc"→"gag"), and the nucleotides 613-615 representing a codon of alanine are substituted by nucleotides representing a codon of leucine ("gcc"→"ctc") in the nucleotide sequence represented by SEQ ID NO:5. In this exemplification, the nucleotides 604-606 after the substitution are not limited and may be nucleotides other than "gag" mentioned above as long as they represent a codon of glutamic acid. Similarly, the nucleotides 613-615 after the substitution are not limited and may be nucleotides other than "ctc" mentioned above as long as they represent a codon of leucine.

As to preparation of such substituted-type DNA mutant, the explanation for the DNA described in (1a) above similarly applies.

The DNA described in (2b) above can be obtained from a cDNA library or a genomic library by a known hybridization method by using DNA containing the nucleotide sequence described in any one of (i) to (iii) (i.e., DNA described in (2a)), DNA composed of a nucleotide sequence complementary to this DNA, or a fragment thereof as a probe. As to the type, procedure and conditions of the hybridization as well as each library, the explanation for the DNA described in (1b) above similarly applies. The DNA to be hybridized has a nucleotide sequence having a homology of preferably at least 40% or more, more preferably 60% or more, still more preferably 90% or more, particularly preferably 95% or more, and most preferably 99% or more to the nucleotide sequence of the DNA described in (2a) above.

Furthermore, in the DNA described in (2b) above, nucleotides are the same as the nucleotides at the substituted site above at the corresponding position.

Herein, the term "substituted site" refers to a site of the nucleotide substitution performed in any one of the nucleotide sequences described in (i) to (iii) contained in the DNA described in (2a) above. Specifically, the term refers to a site of nucleotides (triplet) representing an altered codon resulting from the nucleotide substitution. More specifically, the term "substituted site" refers to the nucleotides 604-606 (provided that the nucleotide sequence is limited to the nucleotide sequences described in (i) and (iii)), and the nucleotides 613-615 (provided that the nucleotide sequence is limited to the nucleotide sequences described in (ii) and (iii)) among the 1,275 nucleotides constituting the nucleotide sequences described in (i) to (iii).

In addition, the "nucleotides" in the phrase "nucleotides corresponding to the nucleotides at the substituted site" refer to nucleotides (triplet) which are positioned to oppose nucleotides (triplet) complementary to the nucleotides at the substituted site in a hybrid prepared by hybridizing the DNA described in (2b) above with a strand complementary to the DNA described in (2a) above. For example, when the nucleotide sequence of the DNA described in (2b) above does not have a mutation such as deletion or addition, as compared to the DNA described in (2a) above (that is, when the lengths (the numbers of nucleotides) of both DNAs are the same), the nucleotides 604-606 and/or the nucleotides 613-615 of the nucleotide sequence of the DNA described in (2b) are the "nucleotides" in the phrase "nucleotides corresponding to the nucleotides at the substituted site".

It is important that the DNA described in (2b) above is DNA that encodes a protein having α-GAL activity. Therefore, for example, nucleotides representing a codon of an amino acid residue which is believed to be important for the binding performance (substrate-binding performance) with an α-galactose residue of a substrate of α-GAL and the catalytic reactivity to the substrate are preferably nucleotides that are not mutated (deleted, substituted or added) from the nucleotide sequences described in (i) to (iii) above. Preferable examples of such nucleotides of the nucleotide sequences described in (i) to (iii) include the nucleotides 124-135 (4 codons), 271-285 (5 codons), 391-423 (11 codons), 490-516 (9 codons), 616-618 (1 codon), 667-702 (12 codons) and 766-804 (13 codons) among the nucleotide sequences described in (i) to (iii) above. Among these nucleotides, the nucleotides 508-510 and 691-693, which represent codons of amino acid residues at a catalytic site, are particularly preferable.

Furthermore, in the DNA described in (2b) above, nucleotides representing a codon of an amino acid residue which is believed to be important for forming a homodimer are also preferably not mutated (deleted, substituted or added) from the nucleotide sequences described in (i) to (iii). Preferable examples of such nucleotides of the nucleotide sequences described in (i) to (iii) include the nucleotides 175-177 and 1,090-1,092 among the nucleotide sequences.

Furthermore, in the DNA described in (2b) above, nucleotides representing a codon of an amino acid residue as an N-type-sugar-chain-binding site are also preferably not mutated (deleted, substituted or added) from the nucleotide sequences described in (i) to (iii) above. Preferable examples of such nucleotides of the nucleotide sequences described in (i) to (iii) above include the nucleotides 412-414, 571-573, 643-645, 1,117-1,119 and 1,195-1,197 among the nucleotide sequences.

A particularly preferable example of the DNA described in (2b) above is DNA composed of a nucleotide sequence which is not completely identical to the nucleotide sequence of the DNA described in (2a) above but which is completely identical to the amino acid sequence after translation (i.e., DNA obtained by performing a silent mutation on the DNA described in (2a) above).

Regarding the above-described gene encoding the protein of the present invention, codons corresponding to each of the amino acids after translation are not particularly limited. Accordingly, the gene encoding the protein of the present invention may contain DNA representing codons which are generally used (preferably, codons whose frequency of use is high) in a mammal such as human after transcription, or DNA representing codons which are generally used (preferably, codons whose frequency of use is high) in, for example, a microorganism such as E. coli or yeast or a plant after transcription.

### <Recombinant vector and transformant>

Generally, in order to express the protein of the present invention, first, a recombinant vector is constructed by introducing the above-described gene of the present invention into an expression vector. In this step, as needed, a transcriptional promoter, SD sequence (in the case where a host is a prokaryotic cell) and Kozak sequence (in the case where a host is a eukaryotic cell) may be linked upstream of the gene to be incorporated into the expression vector, in advance. Alternatively, a terminator may be linked downstream from the gene in advance. Furthermore, an enhancer, a splicing signal, a poly-A addition signal, a selective marker and the like may also be linked in advance. The above elements, such as a transcriptional promoter, required for expressing a gene may be contained in the gene from the beginning. In the case where these elements are contained in the expression vector from the beginning, they may be utilized. The usage embodiments of the elements are not particularly limited.

As a method of incorporating the gene into an expression vector, various types of methods using a known gene recombination technique, for example, a method using a restriction enzyme or a method using a topoisomerase, can be employed. The expression vector is not limited as long as it can retain a gene encoding a protein of the present invention, examples being plasmid DNA, bacteriophage DNA, retrotransposon DNA, a retrovirus vector and artificial chromosome DNA. A vector suitable for a host cell used can appropriately be selected and used.

Subsequently, the constructed recombinant vector is introduced into a host to obtain a transformant, and the transformant is cultured. Thus, the protein of the present invention can be expressed. The term "transformant" used in the present invention refers to a product resulting from introduction of a foreign gene into a host. For example, the transformant includes a product in which a foreign gene is introduced by introducing plasmid DNA or the like into a host (transformation) and a product in which a foreign gene is introduced by infecting a host with a virus or a phage (transduction).

The host is not limited as long as the host can express a protein of the present invention after introduction of the recombinant vector, and can appropriately be selected. Examples of the host include known hosts such as animal cells, e.g., a human cell and a mouse cell, plant cells, bacteria, yeast and the like.

Where an animal cell is used as a host, for example, a human fibroblast, a CHO cell, a simian COS-7 cell, Vero, a mouse L cell, a rat GH3, or a human FL cell is used. Alternatively, insect cells such as an Sf9 cell or an Sf21 cell can also be used.

Where a bacterium is used as a host, for example, E. coli or Bacillus subtilis is used.

Where yeast is used as a host, for example, Saccharomyces cerevisiae or Schizosaccharomyces pombe is used.

Where a plant cell is used as a host, for example, a tobacco BY-2 cell is used.

The method of obtaining a transformant is not limited and can appropriately be selected in consideration of the combination of the types of a host and an expression vector used. Preferable examples of the method include an electroporation method, a lipofection method, a heat shock method, a PEG method, a calcium phosphate method, a DEAE-dextran method, and a method of infecting a virus such as a DNA virus or an RNA virus.

In the resulting transformant, the codon type of a gene contained in the recombinant vector is not limited. The codon type may be identical to or different from the codon type of a host actually used.

### (Method of producing protein)

A protein of the present invention (a protein having α-GAL activity) can be produced, for example, by altering the structure of the active site (in particular, the substrate-binding site) of wild-type α-NAGA so that a substrate of α-GAL can bind thereto. If the substrate of α-GAL can bind to the active site, the substrate can be hydrolyzed via catalysis by the catalytic site of wild-type α-NAGA.

Such a structural alteration is performed as follows. For example, as described above, in the amino acid sequence constituting the active site (substrate-binding site) of wild-type α-NAGA, (i) the 188th serine is substituted by another amino acid such as glutamic acid or aspartic acid, (ii) the 191st alanine is substituted by another amino acid such as leucine, valine, isoleucine, phenylalanine or methionine, or (iii) both the 188th serine and the 191st alanine are substituted as described in (i) and (ii) above by a gene recombination technique. The structural alteration can be achieved by making the structure of the side chain(s) of the amino acid(s) after the replacement from that before the replacement. Consequently, the substrate specificity of wild-type α-NAGA can be altered. In particular, the above-described structural alteration is preferably performed by substituting the 188th serine with glutamic acid and the 191st alanine with leucine. Thereby, the substrate specificity of α-GAL can be imparted to α-NAGA. In the above structural alteration, an amino acid substitution that brings about a significant structural alteration is the substitution of the 191st alanine with leucine or the like. More specifically, the side chain of the amino acid 191 is altered from "-CH₃", i.e., the side chain of alanine, to a bulky side chain, such as "-CH₂-CH(CH₃)-CH₃", i.e., the side chain of leucine. As a result, the space of the active site where the N-acetyl group in a substrate of α-NAGA is to be incorporated is restricted, thereby reducing the binding performance of the protein with the substrate. Instead, the binding performance with a substrate of α-GAL is increased accordingly.

The above-described method for producing a protein of the invention may be carried out, without limitation, by using wild-type α-NAGA containing the signal peptide from wild-type α-GAL, as the wild-type α-NAGA to be subjected to structural alteration, or the method may be carried out via a step of adding (binding) a signal peptide from wild-type α-GAL to an α-NAGA mutant obtained by structural alteration or, if the α-NAGA mutant includes a signal peptide of wild-type α-NAGA, via a step of replacing this signal peptide with a signal peptide from wild-type α-GAL.

Hence, a preferable example of a method for producing the protein of the invention includes a method for producing a protein having α-GAL activity, comprising altering the structure of the active site of wild-type human α-N-acetylgalactosaminidase including a signal peptide from wild-type human α-galactosidase such that a substrate of α-galactosidase can bind thereto.

Moreover, a preferable example of a method for producing the protein of the invention also includes a method for producing a protein having α-GAL activity, comprising adding (binding) a signal peptide from wild-type α-GAL to a protein having the structure of the active site of wild-type α-NAGA altered such that an α-GAL substrate can bind thereto, or comprising replacing a signal peptide moiety of a protein having the structure of the active site of wild-type α-NAGA altered such that an α-GAL substrate can bind thereto with a signal peptide from wild-type α-GAL.

The above-mentioned replacement of the signal peptide moiety may be carried out according to a conventional gene recombination technique by using known nucleotide sequence information and amino acid sequence information on wild-type α-NAGA and wild-type α-GAL.

The protein of the present invention can be produced specifically by a method including a step of culturing the above-described transformant and a step of collecting a protein having α-galactosidase activity from the resulting cultured product. Herein, the term "cultured product" refers to all of a culture supernatant, cultured cells, cultured bacteria, cell debris and bacterial debris. The cultivation of the transformant can be performed in accordance with a general method used for culturing a host. The target protein is accumulated within the cultured product.

As a medium used for the cultivation, any known natural or synthetic medium may be used as long as it contains a carbon source, a nitrogen source, inorganic salts and the like that can be utilized by the host, and as long as the transformant can be cultured efficiently.

In order to prevent loss of a recombinant vector contained in the transformant and loss of a gene encoding a target protein, the cultivation may be performed under a selection pressure. Specifically, in the case where a selective marker is a drug-resistance gene, a corresponding drug can be added to the medium. In the case where a selective marker is an auxotrophic complementary gene, a corresponding nutritional factor can be removed from the medium. For example, in the case where a human fibroblast transduced with a vector containing the G418-resistant gene is cultured, G418 (G418 sulfate) may be added during cultivation, as needed.

In the case where a transformant or the like transformed with an expression vector using an inducible promoter as a promoter is cultured, a favorable inducer (for example, IPTG) may be added to the medium, as needed.

The conditions for culturing the transformant are not particularly limited as long as productivity of the target protein and growth of the host are not interfered. In general, the culture is performed at a temperature in the range of 10°C to 40°C, and preferably in the range of 20°C to 37°C for 5 to 100 hours. The pH can be adjusted using an inorganic or organic acid, an alkaline solution, or the like. Examples of the culture method include solid culture, static culture, shaking culture and aeration-agitation culture.

Once the target protein is produced in bacteria or in cells at the end of cultivation, the target protein can be collected by disrupting the bacteria or the cells. As a method of disrupting the bacteria or the cells, for example, a high-pressure treatment using a French press or a homogenizer, an ultrasonic treatment, a milling treatment using glass beads or the like, an enzyme treatment using lysozyme, cellulase, pectinase, or the like, a freeze-thawing treatment, a treatment with a hypotonic solution, or a bacteriolysis-inducing treatment using a phage can be employed. After the disruption, the disruption residue (which contains an insoluble fraction of a cell extract) of the bacteria or the cells can be removed, as needed. Examples of the method of removing the residue include centrifugal separation and filtration. The efficiency of the removal of residue can be increased using, for example, a flocculant or a filter aid, as needed. The supernatant obtained after the removal of the residue is a soluble fraction of the cell extract, and can be used as a crude protein solution.

When the target protein is produced in bacteria or in cells, alternatively, the bacteria or the cells themselves may be collected by centrifugal separation, membrane separation or the like, and used undisrupted.

On the other hand, when the target protein is produced outside the bacteria or outside the cells, the medium is used directly, or the bacteria or the cells are removed by centrifugal separation, filtration or the like. Subsequently, the target protein is collected from the cultured product by extraction through ammonium sulfate precipitation, as needed, and further isolated and purified by dialysis and chromatography (such as gel filtration, ionexchange chromatography or affinity chromatography).

When the target protein is produced by using bacteria or cells (in or outside the bacteria or cells) as described above, the signal peptide moiety is generally removed upon transportation from the endoplasmic reticulum inside the bacteria or cells to the cytoplasm or upon secretion outside the bacteria or cells, leaving a mature protein to be collected without the signal peptide moiety. The present invention, however, is not limited thereto, and, for example, a protein having two or more (consecutive) signal peptide moieties required for the transportation from the endoplasmic reticulum to the cytoplasm or for the secretion outside the bacteria or cells may be expressed so that a protein having at least one signal peptide moiety left after the transportation or secretion is collected. In this case, if the resulting target protein should be used as an active element of a pharmaceutical composition or the like, the signal peptide moiety may, for example, be cleaved or broken down with an enzyme to use the protein in a form of a mature protein.

The production yield of a protein obtained by culturing a transformant or the like can be determined, for example, by SDS-PAGE (polyacrylamide gel electrophoresis) in terms of unit per culture solution, per wet weight or dry weight of bacteria, per protein in a crude enzyme solution, or the like.

In addition to the protein synthesis system using a transformant described above, a target protein can be produced by using a cell-free protein synthesis system in which no living cells are used.

The cell-free protein synthesis system is a system in which a target protein is synthesized in an artificial container such as a test tube using a cell extract. A cell-free protein synthesis system which can be used also includes a cell-free transcription system in which RNA is synthesized using DNA as a template.

In this case, the cell extract used is preferably derived from the host cell described above. Examples of the cell extract that can be used include extracts derived from eukaryotic cells and prokaryotic cells, more specifically, extracts of a CHO cell, a rabbit reticulocyte, a mouse L-cell, a HeLa cell, wheat germ, budding yeast or E. coli. The cell extract may be, without limitation, concentrated or diluted upon use, or they may be used without further treatment.

The cell extract can be obtained, for example, by ultrafiltration, dialysis or polyethylene glycol (PEG) precipitation.

Alternatively, such cell-free protein synthesis can be performed using a commercially available kit. Examples of the kit include a reagent kit PROTEIOS™ (Toyobo Co., Ltd.), TNT™ System (Promega), a synthesis device PG-Mate™ (Toyobo Co., Ltd.) and RTS (Roche Diagnostics K.K.).

The target protein produced by cell-free protein synthesis can be purified as described above by appropriately selecting means such as chromatography.

According to the method for enhancing stability of the protein of the present invention, the protein is not limited to a particular form. For example, the protein can be in a form of being contained in a composition, in a dry form, or in a form of aqueous solution. The protein of the present invention which serves as an active element in a pharmaceutical composition described later is not limited in terms of state and can be used in a state of a certain type of salt, hydrate, or the like according to need, or can be used in a state of being chemically modified as appropriate in view of preservation stability (in particular, retention of enzyme activity) as a therapeutic agent. An aspect in which the protein is contained in a composition will be described in detail in the section of [3. Pharmaceutical combination for treating Fabry disease].

### [Active site specific chaperone compound]

An active site specific chaperone compound of the present invention is a compound represented by a general formula (I) below 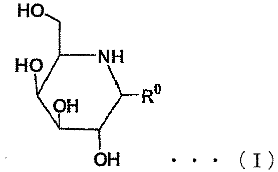 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
the active site specific chaperone compound is a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.

Here, the descriptions of the functional groups in R⁰ of the formula (I) encompass functional groups (e.g., -O-,-SO₃-, -COO-) which correspond to those functional groups and have released hydrogen ions in water to have negative electric charges. Compounds having these functional groups are also encompassed in the compounds represented by the formula (I). The active site specific chaperone compound of the present invention encompasses an active site specific chaperone compound in a form of free base and an active site specific chaperone compound in a form of pharmaceutically acceptable arbitrary salt.

The alkyl group having a carbon number of 1 to 4, the haloalkyl group having a carbon number of 1 to 4, the alkoxy group having a carbon number of 1 to 4, and the hydroxyalkyl group having a carbon number of 1 to 4 can be either in a linear chain form or in a branched chain form.

The alkyl group having a carbon number of 1 to 4 in R⁰ of the formula (I) can be, specifically, a methyl group, an ethyl group, an n-propyl group, an isopropyl group, an n-butyl group, a sec-butyl group, and a tert-butyl group. Among these, an alkyl group having a carbon number of 1 to 3 is preferable, an alkyl group having a carbon number of 1 or 2 is more preferable, and an alkyl group having a carbon number of 1 is further preferable.

A halogen atom in the haloalkyl group or the halogen group can be F, Cl, or Br, and is preferably F or Cl.

The haloalkyl group having a carbon number of 1 to 4 in R⁰ of the formula (I) is a group in which the "halogen atom" in the alkyl group having a carbon number of 1 to 4 is substituted. Examples of the haloalkyl group having a carbon number of 1 to 4 include a trifluoromethyl group, a trichloromethyl group, a difluoromethyl group, a dichloromethyl group, a dibromomethyl group, a fluoromethyl group, a 2,2,2-trifluoroethyl group, a 2,2,2-trichloroethyl group, a 2-bromoethyl group, a 2-chloroethyl group, a 2-fluoroethyl group, a 2-iodoethyl group, a 3-chloropropyl group, a 2,2-dibromoethyl group, a 4-iodobutyl group, a 4-fluorobutyl group, and a 4-chlorobutyl group.

The alkoxy group having a carbon number of 1 to 4 in R⁰ of the formula (I) is a group in which the alkyl group having a carbon number of 1 to 4 is linked with an oxygen atom. Examples of the alkoxy group having a carbon number of 1 to 4 include a methoxy group, an ethoxy group, an n-propoxy group, an isopropoxy group, an n-butoxy group, an isobutoxy group, a sec-butoxy group, and a tert-butoxy group.

The hydroxyalkyl group having a carbon number of 1 to 4 in R⁰ of the formula (I) is a group in which a hydroxyl group in the alkyl group having a carbon number of 1 to 4 is substituted. Examples of the hydroxyalkyl group having a carbon number of 1 to 4 include a hydroxymethyl group, a hydroxyethyl group, a hydroxypropyl group, and a hydroxybutyl group. A favorable example is the hydroxymethyl group.

The cycloalkyl group having a carbon number of 3 or 4 in R⁰ of the formula (I) is a 3-membered or 4-membered saturated or unsaturated cyclic hydrocarbon group. Examples of the cycloalkyl group having a carbon number of 3 or 4 include a cyclopropyl group, a cyclopropylmethyl group, and a cyclobutyl group.

In a certain embodiment, R⁰ of the formula (I) is preferably H, -OH, -SO₃H, or -SO₃⁻, more preferably H or-SO₃H or -SO₃⁻, further more preferably H.

The active site specific chaperone compound in accordance with an embodiment is a competitive inhibitor for the protein of the present invention.

The "competitive inhibitor" for an enzyme refers to a compound which (i) is similar to an enzyme substrate in terms of chemical structure, molecule, geometry, and structure and (ii) binds to an enzyme at a position which is substantially identical with that of the substrate. The active site specific chaperone compound serving as a competitive inhibitor competes with a substrate molecule for the same active site, and increases Km. The competitive inhibition can be reversible. Therefore, a degree of enzyme inhibition depends on a concentration of the competitive inhibitor, a concentration of the substrate, and relative affinities of the inhibitor and the substrate with respect to the active site.

In general, an α-NAGA mutant is more stable at an acidic pH (5.2) (lysosomal condition) than at a neutral pH (7.4). In a case where the α-NAGA mutant is caused to make contact with the active site specific chaperone of the present invention, stability of the enzyme can be improved, as compared with a case where the α-NAGA mutant is not caused to make contact with the active site specific chaperone under both a neutral condition and an acidic condition.

According to an example, the active site specific chaperone preferably raises a denaturation temperature (Tm value) of the α-NAGA mutant.

In a certain embodiment, the active site specific chaperone is 1-deoxy galactonojirimycin (DGJ) in which R⁰ in the structure of the formula (I) is H or is galactostatin bisulfite (GBS) in which R⁰ is -SO₃⁻. In another embodiment, the active site specific chaperone is 1-deoxy galactonojirimycin (DGJ). 1-deoxy galactonojirimycin (DGJ) refers to (2R,3S,4R,5S)-2-(hydroxymethyl)piperdine-3,4,5-triol. When used herein, the terms "1-deoxy galactonojirimycin" and "DGJ" encompass both a free base form thereof and a form of pharmaceutically acceptable arbitrary salt thereof. A hydrochloride of DGJ is known as migalastat hydrochloride.

Note that 1-deoxy galactonojirimycin (DGJ) has the following structure.

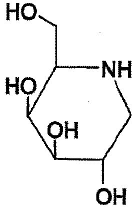

Moreover, galactostatin bisulfite (GBS) has the following structure.

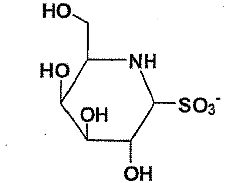

The active site specific chaperone of the present invention has at least one function among the following functions. (1) Aid the protein to have a stable structure; (2) Inhibit proteolysis and facilitate transportation of the protein from ER to an appropriate cellular location; (3) Inhibit aggregation of protein having misfolding (i.e., having an unstable structure); and (4) Enhance protein activity, extend duration of activity, or recover activity.

### [Stability improver for enhancing stability of protein]

As described above, the active site specific chaperone can be used as a stability improver for enhancing stability of a protein. That is, the present invention provides a stability improver for enhancing stability of a protein, the protein being the α-NAGA mutant protein of the present invention, and the stability improver including the active site specific chaperone compound of the present invention.

In the method for enhancing stability of a protein of the present invention, a form of the active site specific chaperone is not limited to a particular one. For example, the active site specific chaperone can be in a form of being contained in a composition, in a dry form, or in a form of aqueous solution. The active site specific chaperone in any of those forms can be used as a stability improver. An aspect in which the active site specific chaperone is contained in a composition will be described in detail in the section of [3. Pharmaceutical combination for treating Fabry disease].

### [Steps in the method of the present invention]

The following description will discuss steps of the method of the present invention in detail.

### (Contact step)

The method for enhancing stability of a protein of the present invention includes a step of causing the protein to make contact with the active site specific chaperone (contact step).

The "stability of a protein" can be determined by checking whether or not the protein retains an appropriate structure and has normal functionality. The structure and functionality of the protein change depending on presence or absence of a substance with which the protein interacts or on environmental conditions (e.g., pH, temperature) under which the protein exists. In view of this, a protein can be said to have higher stability when such structural alteration less occurs and when the functionality less deteriorates.

The degree of stability of a protein can be determined by checking, for example, a degree of activity of the protein itself, change in activity of the protein or in amount of the protein with time, and retention of an appropriate structure.

The method for enhancing stability of a protein can be said as a method for inhibiting deterioration of enzyme activity, a method for maintaining enzyme activity, or a method for improving enzyme activity.

The contact between the protein and the active site specific chaperone in the contact step can be carried out in vivo or in vitro.

An amount of the active site specific chaperone caused to make contact with the protein is an amount necessary for enhancing stability of the protein, and is determined in accordance with, for example, an amount of the protein with which the active site specific chaperone is caused to make contact. A concentration of the active site specific chaperone can be determined by, for example, calculating an IC₅₀ value of the specific chaperone with respect to the enzyme.

In still another embodiment, the method for enhancing stability of a protein of the present invention includes 1) a step of causing the protein of the present invention to make contact with the active site specific chaperone of the present invention (contact step) and 2) a step of measuring stability of the protein after the contact step (measuring step).

### (Measuring step)

The measuring step is carried out after the contact step. The measuring step is a step of measuring stability of the protein after the contact step.

Examples of the method for measuring stability of a protein include measurement of protein activity; measurement of protein structure retention by thermal shift assay, differential scanning calorimetry, or circular dichroism method; and measurement of protein amount by ELISA or western blotting.

In an embodiment, whether or not stability of a protein is enhanced can be confirmed by comparing activity or an expression amount of the protein before carrying out the contact step with activity or an expression amount of the protein after the contact step. A result of analysis on interaction between the active site specific chaperone and the protein can be evaluated with use of a known technique in this field such as plasmon resonance method, differential scanning calorimetry, or circular dichroism method.

The method of the present invention can be applied to a protein to be administered to an individual who needs the protein. That is, the method of the present invention can be used with respect to an α-NAGA mutant that serves as a replacement enzyme for enzyme replacement therapy, and can enhance stability of the α-NAGA mutant in a living body.

### [2. Method for treating Fabry disease]

The present invention provides a method for treating Fabry disease.

### [Enzyme replacement therapy using α-NAGA mutant protein as replacement enzyme, and combination therapy with chaperone therapy using active site specific chaperone]

The method for treating Fabry disease in accordance with an embodiment of the present invention includes administering, to an individual, an effective dose of the protein of the present invention and an effective dose of the active site specific chaperone of the present invention for the protein.

That is, the therapeutic method in accordance with an aspect of the present invention is a method for enhancing stability of an α-NAGA mutant protein during enzyme replacement therapy using the α-NAGA mutant protein. Stability and activity of an α-NAGA mutant protein administered to an individual are enhanced by an active site specific chaperone that has been administered to the same individual. That is, the therapeutic method in accordance with the present embodiment is a combination therapy of the enzyme replacement therapy and the pharmacological chaperone therapy.

In this case, the active site specific chaperone can be administered as an adjuvant for the α-NAGA mutant protein which is a replacement enzyme.

The protein and the active site specific chaperone which are applied are those described in [1. Method for enhancing stability of protein] above. A form of the composition can be any of the forms described in [3. Pharmaceutical combination for treating Fabry disease] below.

The following description will discuss the administration step.

### (Administration step)

The protein and the active site specific chaperone can be simultaneously administered or separately administered. In a case where the protein and the active site specific chaperone are simultaneously administered, the protein and the active site specific chaperone can be contained in different compositions (hereinafter, the composition encompasses a formulation which is a pharmaceutical composition for treating Fabry disease) or can be contained in a single composition. Both of or one of the protein and the active site specific chaperone which are administered can be two or more types of proteins having different structures and/or two or more types of active site specific chaperones having different structures.

### (Administration period)

An administration period is not limited to a particular one, and the administration can be carried out successively or intermittently. For example, the administration can be carried out successively or intermittently (e.g., every 1 to 7 days or every 1 to 14 days) during a period of one month to several months, one year to several years, or several years to several tens of years (e.g., until the individual dies).

### (Administration timing)

In a case where the protein and the active site specific chaperone are contained in different compositions, the protein and the active site specific chaperone can be simultaneously administered. Alternatively, the active site specific chaperone can be administered before or after administration of the protein. For example, in a case where the protein is intravenously administered, the active site specific chaperone can be administered within 0 to 2 hours of the administration of protein, 0 to 2 hours before the administration of protein, or 0 to 2 hours after the administration of protein.

In the administration step, in a case where two or more proteins and/or two or more active site specific chaperones are administered, the two or more types of proteins and/or two or more types of active site specific chaperones can be administered simultaneously or in a mixed form. Alternatively, the two or more types of proteins and/or two or more types of active site specific chaperones can be administered at different points in time. In such a case, it is possible to employ a procedure in which, for example, a certain protein is administered for a certain period, and then a different protein having a different structure is administered for an additional period. Thus, it is possible to change composition components to be administered. A composition of active elements administered during a certain period can be changed in an intended cycle and in an intended order.

### (Route of administration)

A route of administration can be any of intravenous, subcutaneous, intraarterial, intraabdominal, eye drops, intramuscular, transoral, transrectal, transvaginal, intraorbital, intracerebral, intradermal, intracranial, intraspinal, intraventricular, intrathecal, intracisternal, intraarticular, intrapulmonary, intranasal, transmucosal, endermic, and aspiration, and can be oral or parenteral. Note that it is preferable to employ parenteral administration such as intravenous administration, subcutaneous administration, or intraabdominal administration.

In a case where the protein and the active site specific chaperone are prepared as different compositions, the chaperone and the protein can be administered through the same route such as intravenous infusion or can be administered through different routes. For example, it is possible that the protein is administered by intravenous infusion and the active site specific chaperone is administered through a different route such as oral administration.

In a case where the composition containing the protein is administered as a replacement enzyme agent, in general, parenteral administration such as intravenous drip is employed. A formulation that can be used in various types of administration such as parenteral administration can be selected and used as appropriate from among an excipient, a filler, a bulking agent, a binder, a humectant, a disintegrant, a lubricant, a surfactant, a dispersing agent, a buffer, a preservative, a solubilizing agent, an antiseptic, a corrigent, a soothing agent, a stabilizer, an isotonizing agent, and the like which are generally used in production of drugs, and the formulation can be prepared with a routine procedure.

A form of the pharmaceutical composition is not limited to a particular one. In a case where the pharmaceutical composition is a replacement enzyme agent, in general, an intravenously injectable formulation (including intravenous drip) is employed. For example, the pharmaceutical composition can be provided in a form of unit dose ampule or of multiple-dose container.

### (Administration method)

The administration is carried out with a known method, and examples of the administration method include direct injections such as a subcutaneous injection, an intradermal injection, an intravenous injection, an intramuscular injection, and an intraabdominal injection, spray to mucous membranes such as a nasal mucosa, an oral mucosa, a lung mucosa, a vaginal mucosa, and a rectal mucosa, oral administration, intravascular administration, and the like. For example, it is possible to employ a bolus injection of the formulation.

Examples of other useful parenteral delivery systems used in administration include ethylene-vinyl acetate copolymer particles, an osmotic pump, an embedded infusion system, pumping delivery, inclusion cell delivery, liposomal delivery, needle delivery injection, needleless injection, a nebulizer, an aerosol spray, electroporation, and a transdermal patch.

### (Dosage)

A dosage of the protein and the active site specific chaperone of the present invention can be widely determined as appropriate while taking into consideration an age, a body weight, a type of disease, and a condition of disease of an administration target (patient); a route of administration; the number of times of administration; an administration period; and the like, as well as a mixing ratio of active elements in a formulation. In particular, in a case where the protein is administered in a form of pharmaceutical composition serving as a replacement enzyme agent, the number of times of administration of the pharmaceutical composition is preferably about once every two to four weeks, and a dosage (/once) is, for example, an amount in which the protein of the present invention or the like (recombinant enzyme) which is the active element can be administered in an amount of preferably about 0.1 to 10 mg/kg, more preferably about 0.1 to 5 mg/kg, still more preferably about 0.2 to 1 mg/kg, with respect to a body weight of the patient.

### (Administration target)

An individual to be an administration target can be any of animals, and is preferably a vertebrate animal, more preferably a mammal. The mammalian subject can be, in addition to human, domestic animals (such as chicken, pig, horse, goat, sheep, and cattle), pet animals (such as cat, dog, hamster, rabbit, and guinea pig), and experimental animals (such as rodents such as mouse and rat, and monkey). Note, however, that the mammalian subject is particularly preferably human.

For example, an individual is a patient having Fabry disease or has a risk of developing Fabry disease. The phrase "has a risk of developing Fabry disease" intends a case in which one has not developed Fabry disease but is more likely to develop Fabry disease as compared with a healthy person. For example, the phrase encompasses a case in which one has not developed a clinical symptom but has a genetic expression type such as a defect of α-galactosidase gene.

Further, the individual who is the administration target is not limited in age and in gender. An individual in accordance with an embodiment has at least one of the above described clinical symptoms of Fabry disease.

### [Combination therapy employing gene therapy using α-NAGA mutant protein and chaperone therapy using chaperone compound]

The method for treating Fabry disease in accordance with another embodiment of the present invention includes administering, to an individual, an effective dose of a gene encoding the protein of the present invention and an effective dose of the active site specific chaperone of the present invention for the protein.

That is, the therapeutic method in accordance with another aspect of the present invention is a method for enhancing stability of an α-NAGA mutant protein that is expressed in the living body during gene therapy using the α-NAGA mutant gene. That is, the method for treating Fabry disease in accordance with the present embodiment provides a combination therapy in which the above described gene therapy using the gene encoding the protein of the present invention is combined with a chaperone therapy using a chaperone compound.

The protein and the active site specific chaperone which are applied are those described in [1. Method for enhancing stability of protein] above.

A form of the gene that encodes the protein and is administered to an individual can be, for example, a form of composition containing the gene itself, a form of vector in which the gene is incorporated such that the gene can be expressed, or a form of composition that contains the vector. Vectors favorable for administration include an adenovirus vector, an adeno-associated virus vector, a herpesvirus vector, a vaccinia virus vector, a retrovirus vector, a lentivirus vector, and the like. Administration can be efficiently carried out by using any of those virus vectors. Note that it is possible to use a commercially available gene transfer kit (e.g., product name: AdenoExpress, manufactured by Clontech).

In a case where a pharmaceutical composition containing a gene is used in administration, it is possible to employ a technique in which the composition is introduced in a phospholipid endoplasmic reticulum such as a liposome, and then the endoplasmic reticulum is administered. An endoplasmic reticulum holding the gene of the present invention is introduced to a predetermined cell by a lipofection method. Then, the cell thus obtained is, for example, intravenously or intraarterially administered. Alternatively, the cell can be topically administered to a Fabry disease affected organ. For example, in a case where the pharmaceutical composition is administered to an adult, an amount of the pharmaceutical composition is preferably about 0.1 pg/kg to 1000 mg/kg per day, more preferably about 1 pg/kg to 100 mg/kg per day, with respect to a body weight of the patient.

The route of administration, the administration method, the administration form, and the like of the protein described above in [Enzyme replacement therapy using α-NAGA mutant protein as replacement enzyme, and combination therapy with chaperone therapy using active site specific chaperone] are applicable to the administration of the gene and the administration of the active site specific chaperone while replacing the protein (i.e., a component to be administered) with the gene.

### [3. Pharmaceutical combination for treating Fabry disease]

The present invention provides a pharmaceutical combination for treating Fabry disease including (i) the protein of the present invention which has mutations in an amino acid sequence of α-N-acetylgalactosaminidase and has α-galactosidase activity and (ii) the active site specific chaperone of the present invention.

The protein and the active site specific chaperone which are applied are those described in [1. Method for enhancing stability of protein] above.

In the combination, the protein and the active site specific chaperone compound can be in a form of separate substances for using these substances in combination or can be in a form of a single composition in which the protein and the active site specific chaperone compound are contained as components in the same composition. In a case where the combination is in the form of combination of separate substances, for example, the protein and the active site specific chaperone compound can be in forms of separate compositions. Note that other components to be contained in the composition, a dosage form, and the like which will be described later in [Pharmaceutical composition for treating Fabry disease] are applicable in both the form in which the protein and the active site specific chaperone compound are contained in a single composition and the form in which the protein and the active site specific chaperone compound are contained in separate compositions. An active site specific chaperone compound serving as a stability improver for enhancing stability of a protein can be applied to the form of combination of separate substances.

The combination can be suitably used in the method for enhancing stability of a protein of the present invention and in the method for treating Fabry disease. In a specific example of the combination, the protein and the active site specific chaperone compound are prepared as respective medicines which are for treating Fabry disease and to which medicine labels are attached, and these medicines are used in combination. Note that the medicine labels of the respective medicines can indicate that the medicines are used in combination.

### [Pharmaceutical composition for treating Fabry disease]

The present invention provides a pharmaceutical composition for treating Fabry disease which contains one or both of the protein and the active site specific chaperone of the present invention.

In a case where the composition of the present invention is applied to the method for enhancing stability of a protein of the present invention and to the method for treating Fabry disease, as described above, the protein and the active site specific chaperone can be contained in a single composition or can be contained in separate compositions.

The composition containing both the protein and the active site specific chaperone can be, for example, effectively used to simultaneously administer the protein and the active site specific chaperone in the above described therapeutic method.

In a case where the protein and the active site specific chaperone are in forms of separate compositions, the compositions can respectively contain, as other components, identical components or different components.

### [Drug for treating Fabry disease]

An embodiment of the composition which contains the active site specific chaperone compound of the present invention is a drug for treating Fabry disease. The present invention provides the drug for treating Fabry disease which is used in combination with the protein of the present invention and contains the active site specific chaperone compound of the present invention.

### [Pharmaceutical composition as replacement enzyme agent, etc.]

As described above, the protein and the active site specific chaperone of the present invention can exhibit various excellent effects in treatment of Fabry disease, and these can be suitably used as active elements of a pharmaceutical composition for treating Fabry disease (Fabry disease therapeutic agent). That is, the present invention provides a pharmaceutical composition for treating Fabry disease containing the foregoing protein of the present invention. Specifically, the pharmaceutical composition is preferably in a form of replacement enzyme agent that can be used in an enzyme replacement therapy.

In a case where the pharmaceutical composition is used as a replacement enzyme agent, a mixing ratio of the protein of the present invention and types and mixing ratios of the other components can be determined as appropriate in conformity to a publicly known preparation method of a replacement enzyme agent (in particular, an enzyme agent used for enzyme replacement for treating Fabry disease).

According to the present invention, the protein of the present invention (recombinant enzyme) which serves as an active element excels in stability in the blood and is highly efficiently taken in a cell of an affected organ. Therefore, it is possible to achieve an enzyme replacement effect that is equivalent to or excels a conventional technique even with a used amount that is less than that of the conventional technique. Further, the protein of the present invention exhibits an extremely low allergic side effect, and it is therefore possible to significantly reduce physical, mental, and economical burdens to a patient.

Further, the pharmaceutical composition for treating Fabry disease containing the protein and the active site specific chaperone of the present invention brings about a higher therapeutic effect as compared with a conventional composition that does not contain an active site specific chaperone. Therefore, the pharmaceutical composition of the present invention is effective even in a smaller used amount. As such, the pharmaceutical composition for treating Fabry disease of the present invention solves the problem of the conventional enzyme replacement therapy, that is, the pharmaceutical composition of the present invention brings about (i) an effect of maintaining a therapeutic effect by inhibiting production of an antibody to an enzyme due to repetitive administration of recombinant α-GAL and reducing deleterious side reaction and (ii) an effect of maintaining stability in a living body after administration to an individual.

### [Pharmaceutical composition as gene therapeutic drug]

The pharmaceutical composition of the present invention can contain a gene that encodes the protein of the present invention which can exhibit various excellent effects in treatment of Fabry disease as described above.

A form of the gene that is contained in the composition can be, for example, a form of composition containing the gene itself or a form of vector in which the gene is incorporated such that the gene can be expressed.

Specifically, the pharmaceutical composition is preferably in a form of gene therapeutic drug that can be used in a gene therapy.

The pharmaceutical composition of the present invention enhances stability of a protein which is being preserved, being administered in vivo, or being tested in vitro. From this, in a case where the composition is applied to treatment of Fabry disease, it is possible to enhance therapeutic efficacy.

The pharmaceutical composition can contain other components in addition to the protein or the gene of the present invention and the active site specific chaperone of the present invention. Those other components can be pharmaceutically acceptable components (e.g., pharmaceutically acceptable carriers) which would be necessitated depending on a usage (used form) of the pharmaceutical composition. Those other components can be contained as appropriate to an extent that does not impair the effect exhibited by the protein of the present invention or the like. For example, the pharmaceutical composition can further contain substances such as favorable solvent, carrier, excipient, and adjuvant. In a case where the pharmaceutical composition is a pharmaceutical formulation which is suitable for use in injection, the pharmaceutical formulation is preferably an aseptic formulation and has fluidity to an extent of being easily injectable.

### (Carrier)

Examples of a carrier include water, ethanol, polyol (e.g., glycerol, propyleneglycol, polyethyleneglycol), an appropriate mixture thereof, a solvent and a dispersion medium which contain vegetable oil. Adequate fluidity can be maintained, for example, (i) by keeping a necessary particle size by use of a coating such as lecithin (in a case of dispersion) or (ii) by using a surfactant. The action of microorganisms can be prevented, for example, by use of various antibacterial agents and antimycotic agents such as paraben, chlorobutanol, phenol, benzyl alcohol, and sorbic acid.

### (Excipient)

The pharmaceutical composition for treating Fabry disease can contain a pharmaceutically acceptable excipient. Specific examples of the excipient include buffers such as a citrate buffer, a phosphate buffer, an acetate buffer, and a bicarbonate buffer; amino acid, urea, alcohol, ascorbic acid, and phospholipid; proteins such as serum albumin, collagen, and gelatin; EDTA or EGTA and salts such as sodium chloride; liposome; polyvinylpyrrolidone; saccharides such as dextran, mannitol, sorbitol, and glycerol; propyleneglycol and polyethyleneglycol (e.g., PEG-4000, PEG-6000); glycerol; glycine or other amino acid; and lipid.

### (Surfactant)

Examples of a preferable nonionic surfactant include Polysorbate 20, Polysorbate 80, Triton X-100, Triton X-114, Nonidet P-40 octyl-α-glucoside, octyl-β-glucoside, Brij 35, Pluronic, and Tween 20.

### (Filler)

Examples of a filler include glycine, mannitol, albumin, dextran, lactose, microcrystalline cellulose, calcium hydrogen phosphate, and the like.

### (Solvent)

Examples of a solvent for the pharmaceutical composition of the present invention include water, a buffer, and the like. The buffer can be a physiological saline solution, a phosphate buffer, a Ringer's solution, or the like. A solvent that is used for the composition can be a mixture of two or more of the above solvents.

In addition, the solvent can contain a lubricant (e.g., magnesium stearate, talc, or silica), a disintegrant (e.g., cornstarch, potato starch, or sodium starch glycolate), a binder (e.g., pregelatinized starch, polyvinylpyrrolidone, or hydroxypropylmethylcellulose), a humectant (e.g., sodium lauryl sulfate), and/or the like.

### (Protein concentration and active site specific chaperone concentration (active element concentration))

A concentration of the protein in the pharmaceutical composition for treating Fabry disease can be changed in accordance with a purpose of use and an application and is not limited to a particular one. For example, the concentration of the protein can be 0.1 to 10 mg/mL. A concentration of the active site specific chaperone in the pharmaceutical composition for treating Fabry disease can be changed in accordance with the protein concentration and is not limited to a particular one. For example, the concentration of the active site specific chaperone can be 2 mg/mL or less.

### (Dosage form)

A dosage form of the pharmaceutical composition of the present invention is not limited to a particular one, and can be a liquid or solid, or semi-solid or semi-liquid. Alternatively, the dosage form can be a lyophilized formulation. These dosage forms can be easily manufactured based on methods which are known by a person skilled in the art. Examples of the dosage form include a tablet, a pill, powder, a liquid medicine, a suspension, an emulsion, a granule, a capsule, a suppository, an injectable formulation, and the like. The dosage form is preferably the injectable formulation or a dosage form for oral administration such as the tablet. For example, it is possible to employ a formulation for aspiration administration, a formulation for intranasal administration, or a collunarium formulation.

As described above, the method for enhancing stability of a protein of the present invention brings about excellent effects of further enhancing stability of an α-NAGA mutant protein that is effectively used in treatment of Fabry disease and thus enhancing the therapeutic effect. The method for treating Fabry disease and the pharmaceutical composition for treating Fabry disease of the present invention can be effectively used in treatment of Fabry disease.

### [4. Examples of specific aspects of the present invention]

The present invention encompasses any one of the following aspects:
<1> A pharmaceutical combination for treating Fabry disease, the pharmaceutical combination including:
   a protein which has mutations in an amino acid sequence of α-N-acetylgalactosaminidase and has α-galactosidase activity; and
   an active site specific chaperone,
   the protein having mutations in an amino acid sequence of α-N-acetylgalactosaminidase and having α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
      (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
      (b) a protein containing an amino acid sequence in which one or several amino acids other than those located at the substituted sites are deleted, substituted or added in the amino acid sequence described in (a) above, the protein having α-galactosidase activity;
      (c) a protein containing an amino acid sequence represented by SEQ ID NO:6 in which the amino acid 202 is substituted by glutamic acid or aspartic acid while the amino acid 205 is substituted by leucine, valine, or isoleucine; and
      (d) a protein containing an amino acid sequence in which one or several amino acids other than those located at the substituted sites are deleted, substituted or added in the amino acid sequence described in (c) above, the protein having α-galactosidase activity,
   the active site specific chaperone being a compound represented by a general formula (I) below 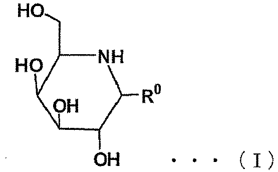 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
   the active site specific chaperone being a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.
<2> The pharmaceutical combination described in <1> in which: in the protein of (a) above, the amino acid 188 is substituted by glutamic acid while the amino acid 191 is substituted by leucine; and
   in the protein of (c) above, the amino acid 202 is substituted by glutamic acid while the amino acid 205 is substituted by leucine.
<3> The pharmaceutical combination described in <1> or <2>, in which the active site specific chaperone is a reversible competitive inhibitor for the protein.
<4> The pharmaceutical combination described in any of <1> through <3>, in which the active site specific chaperone is 1-deoxy galactonojirimycin (DGJ).
<5> A stability improver for enhancing stability of a protein, in which:
   the protein has mutations in an amino acid sequence of α-N-acetylgalactosaminidase and has α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
      (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
      (b) a protein containing an amino acid sequence in which one or several amino acids other than those located at the substituted sites are deleted, substituted or added in the amino acid sequence described in (a) above, the protein having α-galactosidase activity;
      (c) a protein containing an amino acid sequence represented by SEQ ID NO:6 in which the amino acid 202 is substituted by glutamic acid or aspartic acid while the amino acid 205 is substituted by leucine, valine, or isoleucine; and
      (d) a protein containing an amino acid sequence in which one or several amino acids other than those located at the substituted sites are deleted, substituted or added in the amino acid sequence described in (c) above, the protein having α-galactosidase activity,
   the stability improver including a compound selected from the group consisting of α-allo-homonojirimycin, α-galacto-homonojirimycin, α-1-C-butyl-deoxynojirimycin, calystegine A₃, calystegine B₂, N-methyl-calystegine A₃, N-methyl-calystegine B₂, and a compound represented by a general formula (I) below 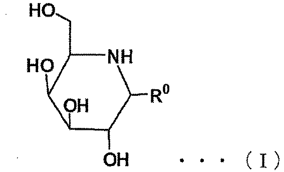 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group.
<6> A drug for treating Fabry disease, in which:
   the drug is used in combination with a protein which has mutations in an amino acid sequence of α-N-acetylgalactosaminidase and has α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
      (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
      (b) a protein containing an amino acid sequence in which one or several amino acids other than those located at the substituted sites are deleted, substituted or added in the amino acid sequence described in (a) above, the protein having α-galactosidase activity;
      (c) a protein containing an amino acid sequence represented by SEQ ID NO:6 in which the amino acid 202 is substituted by glutamic acid or aspartic acid while the amino acid 205 is substituted by leucine, valine, or isoleucine; and
      (d) a protein containing an amino acid sequence in which one or several amino acids other than those located at the substituted sites are deleted, substituted or added in the amino acid sequence described in (c) above, the protein having α-galactosidase activity,
   the drug including a compound selected from the group consisting of α-allo-homonojirimycin, α-galacto-homonojirimycin, α-1-C-butyl-deoxynojirimycin, calystegine A₃, calystegine B₂, N-methyl-calystegine A₃, N-methyl-calystegine B₂, and a compound represented by a general formula (I) below 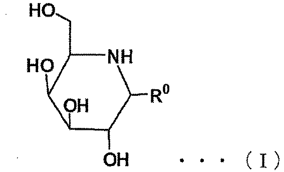 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group.
<7> A method for enhancing stability of a protein including:
   a step of causing the protein to make contact with an active site specific chaperone,
   the protein having mutations in an amino acid sequence of α-N-acetylgalactosaminidase and having α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
      (a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
      (b) a protein containing an amino acid sequence in which one or several amino acids other than those located at the substituted sites are deleted, substituted or added in the amino acid sequence described in (a) above, the protein having α-galactosidase activity;
      (c) a protein containing an amino acid sequence represented by SEQ ID NO:6 in which the amino acid 202 is substituted by glutamic acid or aspartic acid while the amino acid 205 is substituted by leucine, valine, or isoleucine; and
      (d) a protein containing an amino acid sequence in which one or several amino acids other than those located at the substituted sites are deleted, substituted or added in the amino acid sequence described in (c) above, the protein having α-galactosidase activity,
   the active site specific chaperone being a compound represented by a general formula (I) below 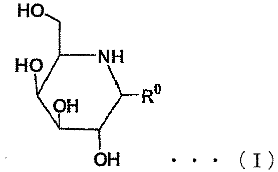 where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
   the active site specific chaperone being a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.
<8> The method described in <7> in which: in the protein of (a) above, the amino acid 188 is substituted by glutamic acid while the amino acid 191 is substituted by leucine; and
   in the protein of (c) above, the amino acid 202 is substituted by glutamic acid while the amino acid 205 is substituted by leucine.
<9> The method described in <7> or <8> in which the active site specific chaperone is a reversible competitive inhibitor for the protein.
<10> The method described in any of <7> through <9> in which the active site specific chaperone is 1-deoxy galactonojirimycin (DGJ).

The present invention will be more specifically described below with reference to Examples. Note, however, that the present invention is not limited to those Examples.

### Examples

### [1. Screening of α-NAGA mutant inhibitor and analysis on inhibition effect]

### Compound

With respect to 30 types of compounds, screening of a compound having an inhibition effect on an α-NAGA mutant was carried out. 1-deoxy galactonojirimycin (DGJ) and galactostatin bisulfite (GBS) were purchased from Toronto Research Chemicals. The other compounds were commercially available compounds which were purchased or prepared by contract synthesis.

### (Screening of inhibitor (1))

The screening of inhibitor was carried out as follows: An inhibitor candidate compound dissolved in purified water or DMSO was added to 20 ng of an α-NAGA mutant (SEQ ID NO:8) and 4 mM 4-methylumbelliferylα-D-galactopyranoside dissolved in 0.1 M phosphate-citrate buffer (pH 4.6) such that a concentration of the inhibitor candidate compound became 0.8, 1, or 400 µM, and the mixture thus obtained was caused to react at 37°C for 30 minutes (the total amount was 50 µl). After the reaction, 950 µl of 0.2 M glycine buffer (pH 10.7) was added to stop the reaction. Then, released 4-methylumbelliferone was measured at an excitation wavelength of 355 nm and at a fluorescence wavelength of 460 nm with use of Wallac 1420 ARVO MX Multilabel Counter. Remaining enzyme activity was calculated based on the assumption that an enzyme activity value obtained in a case where purified water or DMSO is added instead of the inhibitor candidate compound is 100%. As a result, from among the 30 types of compounds, the inhibition effect was seen particularly in cases where DGJ and GBS were added at the high concentration of 400 µM.

### (Screening of inhibitor (2))

Next, with respect to DGJ and GBS which were selected as a result of the first screening, inhibition of enzyme activity of an α-NAGA mutant was measured. Activity was similarly measured with respect to N-C-DGJ, N-DGJ, and N-HE-DGJ, and results thus obtained were used as controls.

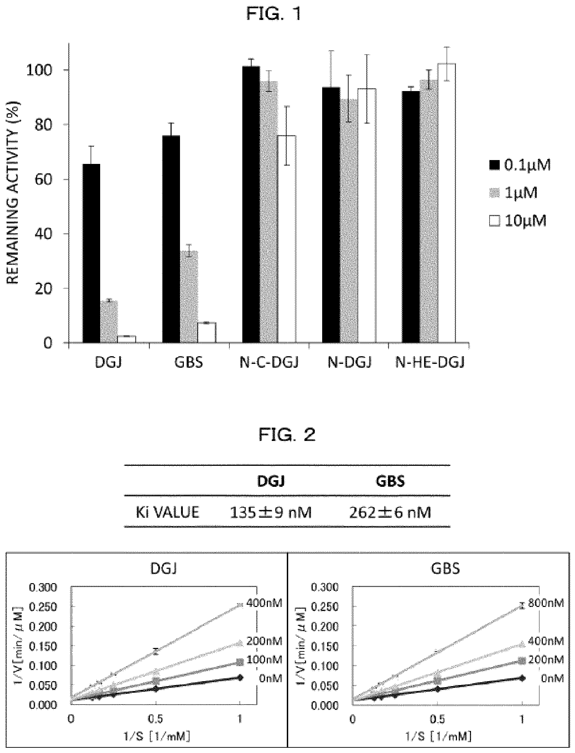

The second screening was carried out as follows: DGJ, GBS, N-C-DGJ, N-DGJ, or N-HE-DGJ dissolved in purified water or DMSO was added to 25 ng of an α-NAGA mutant and 4 mM 4-methylumbelliferylα-D-galactopyranoside dissolved in 0.1 M phosphate-citrate buffer (pH 4.6) such that a concentration of DGJ, GBS, N-C-DGJ, N-DGJ, or N-HE-DGJ became 0.1, 1, or 10 µM, and the mixture thus obtained was caused to react at 37°C for 30 minutes (the total amount was 50 µl). After the reaction was finished, 950 µl of 0.2 M glycine buffer (pH 10.7) was added to stop the reaction. Then, released 4-methylumbelliferone was measured at an excitation wavelength of 355 nm and at a fluorescence wavelength of 460 nm with use of Wallac 1420 ARVO MX Multilabel Counter. Remaining enzyme activity was calculated based on the assumption that an enzyme activity value obtained in a case where purified water or DMSO is added instead of the inhibitor candidate compound is 100%. The results are shown in Fig. 1. Fig. 1 is a view illustrating inhibition of enzyme activity of an α-NAGA mutant by DGJ. DGJ and GBS had the effect of inhibiting activity of an α-NAGA mutant.

### (Calculation of inhibition constant Ki)

Next, an inhibition constant Ki of each of DGJ and GBS was calculated to analyze an inhibition pattern.

Enzymatic analysis was carried out by using, as a reaction liquid, 50 ng of an α-NAGA mutant and 0.1 M phosphate-citrate buffer (pH 4.6) in which a 4-methylumbelliferylα-D-galactopyranoside substrate was dissolved at any of concentrations (1, 2, 4, 6, and 8 mM) (the total amount was 50 µl). In measuring inhibitory activity, reaction was carried out with a reaction liquid which contained DGJ at a concentration of 100, 200, or 400 nM or GBS at a concentration of 200, 400, or 800 nM. After the reaction was carried out at 37°C for 10 minutes, 950 µl of 0.2 M glycine buffer (pH 10.7) was added to stop the reaction. Then, released 4-methylumbelliferone was measured at an excitation wavelength of 355 nm and at a fluorescence wavelength of 460 nm with use of Wallac 1420 ARVO MX Multilabel Counter. From these values, a Lineweaver-Burk plot was prepared and an inhibition constant Ki was calculated from slopes in the plot which correspond to respective concentrations of the inhibitor.

Fig. 2 is a view showing a Lineweaver-Burk plot of enzyme activity of an α-NAGA mutant obtained when DGJ or GBS is used and an inhibition constant Ki calculated from the plot.

From the Ki values, both DGJ and GBS were found to have inhibitory activity by competitive inhibition.

### [2. Analysis on enzyme stabilization effect of α-NAGA mutant inhibitor]

### (Heat stability test carried out by measuring enzyme activity)

Inhibition activity of DGJ with respect to an α-NAGA mutant was tested by a heat stability test.

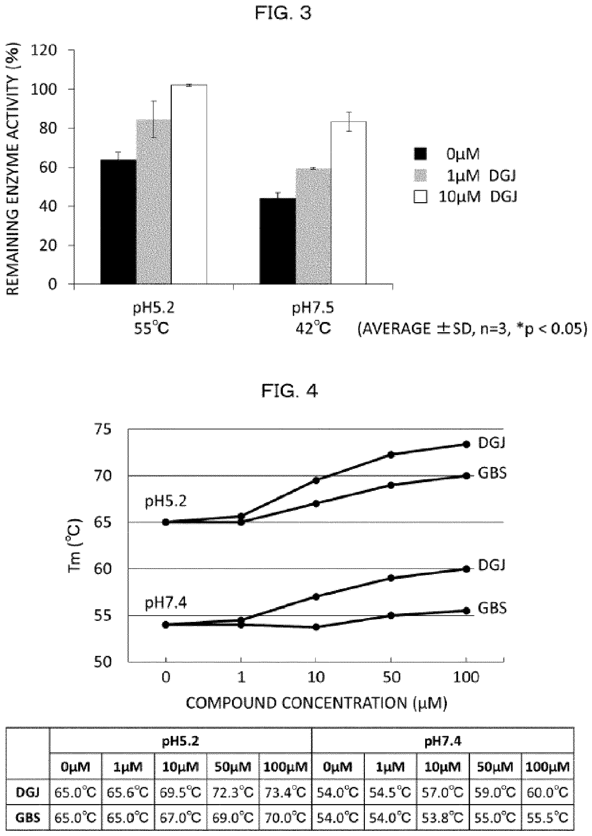

1 µM or 10 µM of DGJ was added to 10 pg/ml of an α-NAGA mutant solution which had been prepared by dissolving an α-NAGA mutant in a 10 mM sodium phosphate buffer (pH 7.5) or a 25 mM sodium acetate buffer (pH 5.2) (the total amount was 100 µl). Then, incubation was carried out at 42°C for the solution using the 10 mM sodium phosphate buffer (pH 7.5) and at 55°C for the solution using the 25 mM sodium acetate buffer (pH 5.2). Then, the incubated solution was sampled by 10 µl at points in time which were respectively 0 and 60 minutes after the start of the incubation. Each of the samples thus obtained was immediately added to 40 µl of a 0.1 M phosphate-citrate buffer (pH 4.6) in which a 5 mM substrate was dissolved, and enzyme activity was measured. Remaining activity is indicated based on an assumption that an enzyme activity value after 0 minutes is 100%. The results are shown in Fig. 3. Fig. 3 is a view showing a result of heat stability test for confirming inhibitory activity of DGJ with respect to an α-NAGA mutant.

Under both the acidic condition of pH 5.2 and the neutral condition of pH 7.5, heat stability of the α-NAGA mutant was improved by the addition of DGJ. The higher improvement in heat stability was seen particularly under the acidic condition.

### (Thermal shift assay)

In regard to DGJ and GBS, Tm values were measured under the acidic condition and the neutral condition.

Analysis of Tm values was carried out by adding 2 µg of an α-NAGA mutant, any of concentrations (1, 10, 50, and 100 µM) of DGJ or GBS, and ×5 SYPRO Orange (Invitrogen) to a 25 mM sodium phosphate buffer (pH 7.4) containing 150 mM NaCl or to a 25 mM sodium acetate buffer (pH 5.2) containing 150 mM NaCl (the total amount was 20 µL). A temperature of the sample was raised from 25°C to 95°C at a rate of 0.5°C/10 seconds with use of qRT-PCR (Bio Rad), and fluorescence intensity was measured over time. Then, a Tm value was calculated with use of CFR Manager software. The results are shown in Fig. 4. Fig. 4 is a view showing results of measuring Tm values obtained when DGJ and GBS are used under an acidic condition and a neutral condition.

### [3. Effect of improving α-NAGA mutant enzyme activity by simultaneous addition of α-NAGA mutant and α-NAGA mutant inhibitor]

### (Simultaneous addition of α-NAGA mutant and DGJ to cultivated fibroblast derived from Fabry disease model mouse)

A cultivated fibroblast derived from a Fabry disease model mouse was cultured at 37°C under a 5% carbon dioxide environment with use of a DMEM medium to which a 10% fetal calf serum and an antibiotic substance had been added. First, concentration dependence of DGJ with respect to uptake efficiency of an α-NAGA mutant was analyzed. The cells were seeded to a 12-well plate at 1.0 × 10⁵ cells/well. The medium was exchanged with an F-10 medium that contained 1 pg/mL of an α-NAGA mutant, any of concentrations (0, 1, 10, 100, and 500 µM) of DGJ, a 10% fetal calf serum, and an antibiotic substance, and cultivation was carried out for 24 hours. The cells were cleaned three times with PBS, then treated with use of trypsin, and then collected. 20 mM MES (pH 6.0) containing Complete Mini (Roche) was added to the collected cells, and the cells were then subjected to an ultrasonic treatment. The homogenate thus obtained was subjected to centrifugal separation under conditions of 4°C and 12,000 rpm, and the obtained supernatant liquid was used as samples for enzyme activity measurement, protein quantification, and Western blotting.

### (Simultaneous addition of α-NAGA mutant and DGJ to cultivated fibroblast derived from patient of Fabry disease)

A cultivated fibroblast derived from a patient of Fabry disease was cultured at 37°C under a 5% carbon dioxide environment with use of an F-10 medium to which a 10% fetal calf serum and an antibiotic substance had been added. First, concentration dependence of DGJ with respect to uptake efficiency of an α-NAGA mutant was analyzed. The cells were seeded to a 12-well plate at 1.0 × 10⁵ cells/well. Then, the medium was exchanged with a medium containing 1 pg/mL of an α-NAGA mutant and any of concentrations (0, 1, 10, and 100 µM) of DGJ, and cultivation was carried out for 24 hours. The cells were cleaned three times with PBS, then treated with use of trypsin, and then collected. 20 mM MES (pH 6.0) containing Complete Mini (Roche) was added to the collected cells, and the cells were then subjected to an ultrasonic treatment. The homogenate thus obtained was subjected to centrifugal separation under conditions of 4°C and 12,000 rpm, and the obtained supernatant liquid was used as samples for enzyme activity measurement, protein quantification, and Western blotting.

Next, in order to confirm time dependence, 1 µg/mL of an α-NAGA mutant and 10 µM of DGJ were added to the cells, and cultivation was carried out for 24 hours, 48 hours, and 72 hours. Then, enzyme activity measurement, protein quantification, and Western blotting were carried out.

### (Enzyme activity measurement)

A solution in which a 5 mM substrate and 117 mM N-acetyl-D-galactosamine (Sigma-Aldrich, St. Louis, MO) were dissolved in a 0.1 M phosphate-citrate buffer (pH 4.6) was used as a substrate solution. 10 µl of the homogenate was added to 40 µl of the substrate solution, and reaction was carried out at 37°C for 30 minutes. After enzyme reaction, 940 µl of a 0.2 M glycine buffer (pH 10.7) was added to stop the reaction. Then, released 4-methylumbelliferone was measured at an excitation wavelength of 355 nm and at a fluorescence wavelength of 460 nm with use of Wallac 1420 ARVO MX Multilabel Counter (Perkin Elmer, Waltham,

### MA).

### (Protein quantification)

Protein quantification was carried out with Micro BCA method (Micro BCA (trademark) Protein Assay Reagent Kit: Pierce) while using a concentration of bovine serum albumin (Albumin standard: Pierce Biotechnology, Inc., Rockford, IL) as a standard. In the measurement, the sample was diluted with MilliQ water, and then an equal quantity of a Micro BCA (trademark) reagent was added, and reaction was carried out at 60°C for one hour. Then, the resultant mixture was cooled to a room temperature, and OD₅₆₀ was measured.

### (Western blotting)

A sample buffer (60 mM Tris-HCl (pH 6.8), 2% SDS, 10% glycerol, 1.5% dithiothreitol) was added to an analysis sample containing a protein, and the resultant sample was heat-treated at 98°C for 5 minutes. The sample was applied to 5-20% bis-trispolyacrylamide gel (e-PAGEL (registered trademark) 5-20% Bis-Tris Gel; ATTO), and electrophoresis was carried out for 83 minutes at a constant current with use of a Tris-Glycine SDS running buffer (25 mM Tris, 92 mM Glycine, 0.1% SDS). Then, the sample was transferred to a PVDF membrane with use of TE77PWR (GE Healthcare). After the transcription was finished, the PVDF membrane was blocked for 60 minutes with use of a blocking buffer (tris-hydrochloric acid buffered saline (50 mM tris-hydrochloric acid buffer (pH 7.4)) containing 5% skim milk and 0.1% Tween 20, and 100 mM sodium chloride), and an anti-human NAGA rabbit polyclonal antibody (primary antibody) diluted 500 times with a blocking buffer was added. Then, reaction was carried out overnight at 4°C. The PVDF membrane which had been reacted with the primary antibody was cleaned three times (5 minutes each) with use of a tris-hydrochloric acid buffered saline containing 0.1% Tween 20. Then, a goat anti-rabbit IgG peroxidase-labeled antibody (HRP labeled secondary antibody) (GE Healthcare) diluted 5000 times with a blocking buffer was added, and reaction was carried out for one hour at a room temperature. The PVDF membrane which had been reacted with the secondary antibody was cleaned three times (5 minutes each) with use of a tris-hydrochloric acid buffered saline containing 0.1% Tween 20. Then, a coloring reagent EzWestLumi plus (ATTO) was added, and reaction was carried out for one minute at a room temperature. Then, luminescence was detected with use of ImageQuant (GE Healthcare).

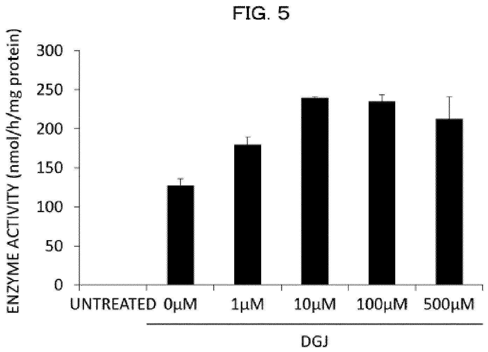

In detection of GAPDH, an anti-GAPDH mouse monoclonal antibody (MBL) diluted 5000 times with a blocking buffer was used as a primary antibody, and a goat anti-mouse IgG peroxidase-labeled antibody (GE Healthcare) diluted 5000 times with a blocking buffer was used as a secondary antibody. The results are shown in Fig. 5. Fig. 5 is a view showing enzyme activity of an α-NAGA mutant which is measured when the α-NAGA mutant has been added simultaneously with various concentrations of DGJ to a cultivated fibroblast derived from a Fabry disease model mouse. Fig. 6 is a view showing enzyme activity of an α-NAGA mutant and enzyme uptake amounts of the α-NAGA mutant which are measured when the α-NAGA mutant has been added simultaneously with various concentrations of DGJ to a cultivated fibroblast derived from a patient of Fabry disease (in Fig. 6, "m. NAGA" represents an α-NAGA mutant, "Wild" represents a cultivated fibroblast derived from a healthy person as a control, and "Fabry" represents a cultivated fibroblast derived from a patient of Fabry disease). (a) of Fig. 6 shows enzyme activity of an α-NAGA mutant which is measured when the α-NAGA mutant has been added simultaneously with various concentrations of DGJ to a cultivated fibroblast derived from a patient of Fabry disease. (b) of Fig. 6 shows an enzyme uptake amount of the α-NAGA mutant which is measured when the α-NAGA mutant has been added simultaneously with various concentrations of DGJ to a cultivated fibroblast derived from a patient of Fabry disease. Fig. 7 is a view showing changes in enzyme activity and in enzyme uptake amount with time of an α-NAGA mutant which are measured when the α-NAGA mutant has been added simultaneously with DGJ to a cultivated fibroblast derived from a patient of Fabry disease (in Fig. 7, "m. NAGA" represents an α-NAGA mutant). (a) of Fig. 7 shows a change in enzyme activity with time of an α-NAGA mutant which is measured when the α-NAGA mutant has been added simultaneously with DGJ to a cultivated fibroblast derived from a patient of Fabry disease, and (b) of Fig. 7 shows a change in enzyme uptake amount with time of the α-NAGA mutant which is measured when the α-NAGA mutant has been added simultaneously with DGJ to a cultivated fibroblast derived from a patient of Fabry disease.

As such, the α-NAGA mutant enzyme activity was improved by simultaneously adding the α-NAGA mutant and DGJ. Moreover, an amount of protein of the α-NAGA mutant which had been taken in a cell was confirmed to be increased by the simultaneous addition of DGJ, as compared with a case where DGJ was not added.

### Industrial Applicability

The present invention can be used in treatment of Fabry disease.

## Claims

1. A pharmaceutical combination for treating Fabry disease, said pharmaceutical combination comprising:
a protein which has mutations in an amino acid sequence of α-N-acetylgalactosaminidase and has α-galactosidase activity; and
an active site specific chaperone,
the protein having mutations in an amino acid sequence of α-N-acetylgalactosaminidase and having α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
(a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
(b) a protein containing an amino acid sequence in which one or several amino acids other than those located at the substituted sites are deleted, substituted or added in the amino acid sequence recited in (a) above, the protein having α-galactosidase activity;
(c) a protein containing an amino acid sequence represented by SEQ ID NO:6 in which the amino acid 202 is substituted by glutamic acid or aspartic acid while the amino acid 205 is substituted by leucine, valine, or isoleucine; and
(d) a protein containing an amino acid sequence in which one or several amino acids other than those located at the substituted sites are deleted, substituted or added in the amino acid sequence recited in (c) above, the protein having α-galactosidase activity,
the active site specific chaperone being a compound represented by a general formula (I) below 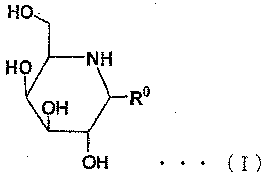
where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
the active site specific chaperone being a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.

2. The pharmaceutical combination as set forth in claim 1, wherein:
in the protein of (a) above, the amino acid 188 is substituted by glutamic acid while the amino acid 191 is substituted by leucine; and
in the protein of (c) above, the amino acid 202 is substituted by glutamic acid while the amino acid 205 is substituted by leucine.

3. The pharmaceutical combination as set forth in claim 1 or 2, wherein the active site specific chaperone is a reversible competitive inhibitor for the protein.

4. The pharmaceutical combination as set forth in any one of claims 1 through 3, wherein the active site specific chaperone is 1-deoxy galactonojirimycin (DGJ).

5. A stability improver for enhancing stability of a protein, wherein:
the protein has mutations in an amino acid sequence of α-N-acetylgalactosaminidase and has α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
(a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
(b) a protein containing an amino acid sequence in which one or several amino acids other than those located at the substituted sites are deleted, substituted or added in the amino acid sequence recited in (a) above, the protein having α-galactosidase activity;
(c) a protein containing an amino acid sequence represented by SEQ ID NO:6 in which the amino acid 202 is substituted by glutamic acid or aspartic acid while the amino acid 205 is substituted by leucine, valine, or isoleucine; and
(d) a protein containing an amino acid sequence in which one or several amino acids other than those located at the substituted sites are deleted, substituted or added in the amino acid sequence recited in (c) above, the protein having α-galactosidase activity,
said stability improver including a compound selected from the group consisting of α-allo-homonojirimycin, α-galacto-homonojirimycin, α-1-C-butyl-deoxynojirimycin, calystegine A₃, calystegine B₂, N-methyl-calystegine A₃, N-methyl-calystegine B₂, and a compound represented by a general formula (I) below 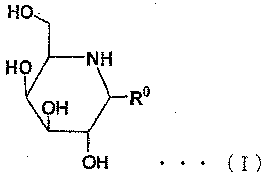
where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group.

6. A drug for treating Fabry disease, wherein:
said drug is used in combination with a protein which has mutations in an amino acid sequence of α-N-acetylgalactosaminidase and has α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
(a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
(b) a protein containing an amino acid sequence in which one or several amino acids other than those located at the substituted sites are deleted, substituted or added in the amino acid sequence recited in (a) above, the protein having α-galactosidase activity;
(c) a protein containing an amino acid sequence represented by SEQ ID NO:6 in which the amino acid 202 is substituted by glutamic acid or aspartic acid while the amino acid 205 is substituted by leucine, valine, or isoleucine; and
(d) a protein containing an amino acid sequence in which one or several amino acids other than those located at the substituted sites are deleted, substituted or added in the amino acid sequence recited in (c) above, the protein having α-galactosidase activity,
said drug comprising a compound selected from the group consisting of α-allo-homonojirimycin, α-galacto-homonojirimycin, α-1-C-butyl-deoxynojirimycin, calystegine A₃, calystegine B₂, N-methyl-calystegine A₃, N-methyl-calystegine B₂, and a compound represented by a general formula (I) below 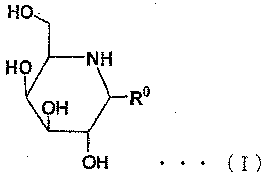
where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group.

7. A method for enhancing stability of a protein, said method comprising the step of:
causing the protein to make contact with an active site specific chaperone,
the protein having mutations in an amino acid sequence of α-N-acetylgalactosaminidase and having α-galactosidase activity, the protein being any of the following (a) through (d) (except for a protein containing an amino acid sequence represented by SEQ ID NO:2 in which the amino acid 188 is substituted by glutamic acid or aspartic acid while the amino acid 191 is substituted by leucine, valine, or isoleucine),
(a) a protein containing an amino acid sequence composed of the amino acids 18-411 of the amino acid sequence represented by SEQ ID NO:2 in which the amino acid 188 is substituted by glutamic acid or aspartic acid while the amino acid 191 is substituted by leucine, valine, or isoleucine;
(b) a protein containing an amino acid sequence in which one or several amino acids other than those located at the substituted sites are deleted, substituted or added in the amino acid sequence recited in (a) above, the protein having α-galactosidase activity;
(c) a protein containing an amino acid sequence represented by SEQ ID NO:6 in which the amino acid 202 is substituted by glutamic acid or aspartic acid while the amino acid 205 is substituted by leucine, valine, or isoleucine; and
(d) a protein containing an amino acid sequence in which one or several amino acids other than those located at the substituted sites are deleted, substituted or added in the amino acid sequence recited in (c) above, the protein having α-galactosidase activity,
the active site specific chaperone being a compound represented by a general formula (I) below 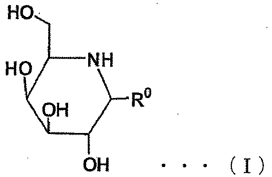
where R⁰ represents H, -OH, -SO₃H, -SO₃⁻, -COOH, an alkyl group having a carbon number of 1 to 4, a haloalkyl group having a carbon number of 1 to 4, an alkoxy group having a carbon number of 1 to 4, a hydroxyalkyl group having a carbon number of 1 to 4, a cycloalkyl group having a carbon number of 3 or 4, or a halogen group, or
the active site specific chaperone being a compound selected from the group consisting of α-allo-homonojirimycin, α-galacto-homonojirimycin, α-1-C-butyl-deoxynojirimycin, calystegine A₃, calystegine B₂, N-methyl-calystegine A₃, and N-methyl-calystegine B₂.

8. The method as set forth in claim 7, wherein:
in the protein of (a) above, the amino acid 188 is substituted by glutamic acid while the amino acid 191 is substituted by leucine; and
in the protein of (c) above, the amino acid 202 is substituted by glutamic acid while the amino acid 205 is substituted by leucine.

9. The method as set forth in claim 7 or 8, wherein the active site specific chaperone is a reversible competitive inhibitor for the protein.

10. The method as set forth in any one of claims 7 through 9, wherein the active site specific chaperone is 1-deoxy galactonojirimycin (DGJ).


SEQUENCE LISTING

<110>  MEIJI PHARMACEUTICAL UNIVERSITY

<120>  Combination of Medicaments for Treatment of Fabry disease and Use
       Thereof

<130>  MPU18158

<150>  JP2017-117266
<151>  2017-06-14

<160>  12

<170>  PatentIn version 3.5

<210>  1
<211>  1236
<212>  DNA
<213>  Homo sapiens

<400>  1
atgctgctga agacagtgct cttgctggga catgtggccc aggtgctgat gctggacaat      60

gggctcctgc agacaccacc catgggctgg ctggcctggg aacgcttccg ctgcaacatt     120

aactgtgatg aggacccaaa gaactgcata agtgaacagc tcttcatgga gatggctgac     180

cggatggcac aggatggatg gcgggacatg ggctacacat acctaaacat tgatgactgc     240

tggatcggcg gtcgcgatgc cagtggccgc ctgatgccag atcccaagcg cttccctcat     300

ggcattcctt tcctggctga ctacgttcac tccctgggcc tgaagttggg tatctacgcg     360

gacatgggca acttcacctg catgggttac ccaggcacca cactggacaa ggtggtccag     420

gatgctcaga ccttcgccga gtggaaggta gacatgctca agctggatgg ctgcttctcc     480

acccccgagg agcgggccca ggggtacccc aagatggctg ctgccctgaa tgccacaggc     540

cgccccatcg ccttctcctg cagctggcca gcctatgaag gcggcctccc cccaagggtg     600

aactacagtc tgctggcgga catctgcaac ctctggcgta actatgatga catccaggac     660

tcctggtgga gcgtgctctc catcctgaat tggttcgtgg agcaccagga catactgcag     720

ccagtggccg gccctgggca ctggaatgac cctgacatgc tgctcattgg gaactttggt     780

ctcagcttag agcaatcccg ggcccagatg gccctgtgga cggtgctggc agcccccctc     840

ttgatgtcca cagacctgcg taccatctcc gcccagaaca tggacattct gcagaatcca     900

ctcatgatca aaatcaacca ggatccctta ggcatccagg gacgcaggat tcacaaggaa     960

aaatctctca tcgaagtgta catgcggcct ctgtccaaca aggctagcgc cttagtcttc    1020

ttcagctgca ggaccgatat gccttatcgc taccactcct cccttggcca gctgaacttc    1080

accgggtctg tgatatatga ggcccaggac gtctactcag gtgacatcat cagtggcctc    1140

cgagatgaaa ccaacttcac agtgatcatc aacccttcag gggtagtgat gtggtacctg     1200

tatcccatca agaacctgga gatgtcccag cagtga                              1236

<210>  2
<211>  411
<212>  PRT
<213>  Homo sapiens

<400>  2

Met Leu Leu Lys Thr Val Leu Leu Leu Gly His Val Ala Gln Val Leu
1               5                   10                  15

Met Leu Asp Asn Gly Leu Leu Gln Thr Pro Pro Met Gly Trp Leu Ala
            20                  25                  30

Trp Glu Arg Phe Arg Cys Asn Ile Asn Cys Asp Glu Asp Pro Lys Asn
        35                  40                  45

Cys Ile Ser Glu Gln Leu Phe Met Glu Met Ala Asp Arg Met Ala Gln
    50                  55                  60

Asp Gly Trp Arg Asp Met Gly Tyr Thr Tyr Leu Asn Ile Asp Asp Cys
65                  70                  75                  80

Trp Ile Gly Gly Arg Asp Ala Ser Gly Arg Leu Met Pro Asp Pro Lys
                85                  90                  95

Arg Phe Pro His Gly Ile Pro Phe Leu Ala Asp Tyr Val His Ser Leu
            100                 105                 110

Gly Leu Lys Leu Gly Ile Tyr Ala Asp Met Gly Asn Phe Thr Cys Met
        115                 120                 125

Gly Tyr Pro Gly Thr Thr Leu Asp Lys Val Val Gln Asp Ala Gln Thr
    130                 135                 140

Phe Ala Glu Trp Lys Val Asp Met Leu Lys Leu Asp Gly Cys Phe Ser
145                 150                 155                 160

Thr Pro Glu Glu Arg Ala Gln Gly Tyr Pro Lys Met Ala Ala Ala Leu
                165                 170                 175

Asn Ala Thr Gly Arg Pro Ile Ala Phe Ser Cys Ser Trp Pro Ala Tyr
            180                 185                 190

Glu Gly Gly Leu Pro Pro Arg Val Asn Tyr Ser Leu Leu Ala Asp Ile
        195                 200                 205

Cys Asn Leu Trp Arg Asn Tyr Asp Asp Ile Gln Asp Ser Trp Trp Ser
    210                 215                 220

Val Leu Ser Ile Leu Asn Trp Phe Val Glu His Gln Asp Ile Leu Gln
225                 230                 235                 240

Pro Val Ala Gly Pro Gly His Trp Asn Asp Pro Asp Met Leu Leu Ile
                245                 250                 255

Gly Asn Phe Gly Leu Ser Leu Glu Gln Ser Arg Ala Gln Met Ala Leu
            260                 265                 270

Trp Thr Val Leu Ala Ala Pro Leu Leu Met Ser Thr Asp Leu Arg Thr
        275                 280                 285

Ile Ser Ala Gln Asn Met Asp Ile Leu Gln Asn Pro Leu Met Ile Lys
    290                 295                 300

Ile Asn Gln Asp Pro Leu Gly Ile Gln Gly Arg Arg Ile His Lys Glu
305                 310                 315                 320

Lys Ser Leu Ile Glu Val Tyr Met Arg Pro Leu Ser Asn Lys Ala Ser
                325                 330                 335

Ala Leu Val Phe Phe Ser Cys Arg Thr Asp Met Pro Tyr Arg Tyr His
            340                 345                 350

Ser Ser Leu Gly Gln Leu Asn Phe Thr Gly Ser Val Ile Tyr Glu Ala
        355                 360                 365

Gln Asp Val Tyr Ser Gly Asp Ile Ile Ser Gly Leu Arg Asp Glu Thr
    370                 375                 380

Asn Phe Thr Val Ile Ile Asn Pro Ser Gly Val Val Met Trp Tyr Leu
385                 390                 395                 400

Tyr Pro Ile Lys Asn Leu Glu Met Ser Gln Gln
                405                 410

<210>  3
<211>  1236
<212>  DNA
<213>  Artificial

<220>
<223>  recombinant DNA

<400>  3
   atgctgctga agacagtgct cttgctggga catgtggccc aggtgctgat gctggacaat        60
   gggctcctgc agacaccacc catgggctgg ctggcctggg aacgcttccg ctgcaacatt       120
   aactgtgatg aggacccaaa gaactgcata agtgaacagc tcttcatgga gatggctgac 180
   cggatggcac aggatggatg gcgggacatg ggctacacat acctaaacat tgatgactgc 240
   tggatcggcg gtcgcgatgc cagtggccgc ctgatgccag atcccaagcg cttccctcat 300
   ggcattcctt tcctggctga ctacgttcac tccctgggcc tgaagttggg tatctacgcg 360
   gacatgggca acttcacctg catgggttac ccaggcacca cactggacaa ggtggtccag 420
   gatgctcaga ccttcgccga gtggaaggta gacatgctca agctggatgg ctgcttctcc 480
   acccccgagg agcgggccca ggggtacccc aagatggctg ctgccctgaa tgccacaggc 540
   cgccccatcg ccttctcctg cgagtggcca ctctatgaag gcggcctccc cccaagggtg 600
   aactacagtc tgctggcgga catctgcaac ctctggcgta actatgatga catccaggac 660
   tcctggtgga gcgtgctctc catcctgaat tggttcgtgg agcaccagga catactgcag 720
   ccagtggccg gccctgggca ctggaatgac cctgacatgc tgctcattgg gaactttggt 780
   ctcagcttag agcaatcccg ggcccagatg gccctgtgga cggtgctggc agcccccctc 840
   ttgatgtcca cagacctgcg taccatctcc gcccagaaca tggacattct gcagaatcca 900
   ctcatgatca aaatcaacca ggatccctta ggcatccagg gacgcaggat tcacaaggaa 960
   aaatctctca tcgaagtgta catgcggcct ctgtccaaca aggctagcgc cttagtcttc 1020
   ttcagctgca ggaccgatat gccttatcgc taccactcct cccttggcca gctgaacttc 1080
   accgggtctg tgatatatga ggcccaggac gtctactcag gtgacatcat cagtggcctc 1140
   cgagatgaaa ccaacttcac agtgatcatc aacccttcag gggtagtgat gtggtacctg 1200
   tatcccatca agaacctgga gatgtcccag cagtga 1236

<210> 4
<211> 411
<212> PRT
<213> Artificial

<220>
<223> recombinant protein

<400> 4

<210>  5
<211>  1278
<212>  DNA
<213>  Artificial

<220>
<223>  recombinant DNA

<400>  5
atgcagctga ggaacccaga actacatctg ggctgcgcgc ttgcgcttcg cttcctggcc      60

ctcgtttcct gggacatccc tggggctaga gcactggaca atgggctcct gcagacacca     120

cccatgggct ggctggcctg ggaacgcttc cgctgcaaca ttaactgtga tgaggaccca     180

aagaactgca taagtgaaca gctcttcatg gagatggctg accggatggc acaggatgga     240

tggcgggaca tgggctacac atacctaaac attgatgact gctggatcgg cggtcgcgat     300

gccagtggcc gcctgatgcc agatcccaag cgcttccctc atggcattcc tttcctggct     360

gactacgttc actccctggg cctgaagttg ggtatctacg cggacatggg caacttcacc     420

tgcatgggtt acccaggcac cacactggac aaggtggtcc aggatgctca gaccttcgcc     480

gagtggaagg tagacatgct caagctggat ggctgcttct ccacccccga ggagcgggcc     540

caggggtacc ccaagatggc tgctgccctg aatgccacag gccgccccat cgccttctcc     600

tgcagctggc cagcctatga aggcggcctc cccccaaggg tgaactacag tctgctggcg     660

gacatctgca acctctggcg taactatgat gacatccagg actcctggtg gagcgtgctc     720

tccatcctga attggttcgt ggagcaccag gacatactgc agccagtggc cggccctggg     780

cactggaatg accctgacat gctgctcatt gggaactttg gtctcagctt agagcaatcc     840

cgggcccaga tggccctgtg gacggtgctg gcagcccccc tcttgatgtc cacagacctg 900

cgtaccatct ccgcccagaa catggacatt ctgcagaatc cactcatgat caaaatcaac 960

caggatccct taggcatcca gggacgcagg attcacaagg aaaaatctct catcgaagtg 1020

tacatgcggc ctctgtccaa caaggctagc gccttagtct tcttcagctg caggaccgat 1080

atgccttatc gctaccactc ctcccttggc cagctgaact tcaccgggtc tgtgatatat 1140

gaggcccagg acgtctactc aggtgacatc atcagtggcc tccgagatga aaccaacttc 1200

acagtgatca tcaacccttc aggggtagtg atgtggtacc tgtatcccat caagaacctg 1260

gagatgtccc agcagtga 1278

<210> 6
<211> 425
<212> PRT
<213> Artificial

<220>
<223> recombinant protein

<400> 6

Met Gln Leu Arg Asn Pro Glu Leu His Leu Gly Cys Ala Leu Ala Leu
1               5                   10                  15

Arg Phe Leu Ala Leu Val Ser Trp Asp Ile Pro Gly Ala Arg Ala Leu
            20                  25                  30

Asp Asn Gly Leu Leu Gln Thr Pro Pro Met Gly Trp Leu Ala Trp Glu
        35                  40                  45

Arg Phe Arg Cys Asn Ile Asn Cys Asp Glu Asp Pro Lys Asn Cys Ile
    50                  55                  60

Ser Glu Gln Leu Phe Met Glu Met Ala Asp Arg Met Ala Gln Asp Gly
65                  70                  75                  80

Trp Arg Asp Met Gly Tyr Thr Tyr Leu Asn Ile Asp Asp Cys Trp Ile
                85                  90                  95

Gly Gly Arg Asp Ala Ser Gly Arg Leu Met Pro Asp Pro Lys Arg Phe
            100                 105                 110

Pro His Gly Ile Pro Phe Leu Ala Asp Tyr Val His Ser Leu Gly Leu
        115                 120                 125

Lys Leu Gly Ile Tyr Ala Asp Met Gly Asn Phe Thr Cys Met Gly Tyr
    130                 135                 140

Pro Gly Thr Thr Leu Asp Lys Val Val Gln Asp Ala Gln Thr Phe Ala
145                 150                 155                 160

Glu Trp Lys Val Asp Met Leu Lys Leu Asp Gly Cys Phe Ser Thr Pro
                165                 170                 175

Glu Glu Arg Ala Gln Gly Tyr Pro Lys Met Ala Ala Ala Leu Asn Ala
            180                 185                 190

Thr Gly Arg Pro Ile Ala Phe Ser Cys Ser Trp Pro Ala Tyr Glu Gly
        195                 200                 205

Gly Leu Pro Pro Arg Val Asn Tyr Ser Leu Leu Ala Asp Ile Cys Asn
    210                 215                 220

Leu Trp Arg Asn Tyr Asp Asp Ile Gln Asp Ser Trp Trp Ser Val Leu
225                 230                 235                 240

Ser Ile Leu Asn Trp Phe Val Glu His Gln Asp Ile Leu Gln Pro Val
                245                 250                 255

Ala Gly Pro Gly His Trp Asn Asp Pro Asp Met Leu Leu Ile Gly Asn
            260                 265                 270

Phe Gly Leu Ser Leu Glu Gln Ser Arg Ala Gln Met Ala Leu Trp Thr
        275                 280                 285

Val Leu Ala Ala Pro Leu Leu Met Ser Thr Asp Leu Arg Thr Ile Ser
    290                 295                 300

Ala Gln Asn Met Asp Ile Leu Gln Asn Pro Leu Met Ile Lys Ile Asn
305                 310                 315                 320

Gln Asp Pro Leu Gly Ile Gln Gly Arg Arg Ile His Lys Glu Lys Ser
                325                 330                 335

Leu Ile Glu Val Tyr Met Arg Pro Leu Ser Asn Lys Ala Ser Ala Leu
            340                 345                 350

Val Phe Phe Ser Cys Arg Thr Asp Met Pro Tyr Arg Tyr His Ser Ser
        355                 360                 365

Leu Gly Gln Leu Asn Phe Thr Gly Ser Val Ile Tyr Glu Ala Gln Asp
    370                 375                 380

Val Tyr Ser Gly Asp Ile Ile Ser Gly Leu Arg Asp Glu Thr Asn Phe

385                 390                 395                 400

Thr Val Ile Ile Asn Pro Ser Gly Val Val Met Trp Tyr Leu Tyr Pro
                405                 410                 415

Ile Lys Asn Leu Glu Met Ser Gln Gln
            420                 425

<210>  7
<211>  1278
<212>  DNA
<213>  Artificial

<220>
<223>  recombinant DNA

<400>  7
atgcagctga ggaacccaga actacatctg ggctgcgcgc ttgcgcttcg cttcctggcc      60

ctcgtttcct gggacatccc tggggctaga gcactggaca atgggctcct gcagacacca     120

cccatgggct ggctggcctg ggaacgcttc cgctgcaaca ttaactgtga tgaggaccca     180

aagaactgca taagtgaaca gctcttcatg gagatggctg accggatggc acaggatgga     240

tggcgggaca tgggctacac atacctaaac attgatgact gctggatcgg cggtcgcgat     300

gccagtggcc gcctgatgcc agatcccaag cgcttccctc atggcattcc tttcctggct     360

gactacgttc actccctggg cctgaagttg ggtatctacg cggacatggg caacttcacc     420

tgcatgggtt acccaggcac cacactggac aaggtggtcc aggatgctca gaccttcgcc     480

gagtggaagg tagacatgct caagctggat ggctgcttct ccacccccga ggagcgggcc     540

caggggtacc ccaagatggc tgctgccctg aatgccacag gccgccccat cgccttctcc     600

tgcgagtggc cactctatga aggcggcctc cccccaaggg tgaactacag tctgctggcg     660

gacatctgca acctctggcg taactatgat gacatccagg actcctggtg gagcgtgctc     720

tccatcctga attggttcgt ggagcaccag gacatactgc agccagtggc cggccctggg     780

cactggaatg accctgacat gctgctcatt gggaactttg gtctcagctt agagcaatcc     840

cgggcccaga tggccctgtg gacggtgctg gcagcccccc tcttgatgtc cacagacctg     900

cgtaccatct ccgcccagaa catggacatt ctgcagaatc cactcatgat caaaatcaac     960

caggatccct taggcatcca gggacgcagg attcacaagg aaaaatctct catcgaagtg    1020

tacatgcggc ctctgtccaa caaggctagc gccttagtct tcttcagctg caggaccgat    1080

atgccttatc gctaccactc ctcccttggc cagctgaact tcaccgggtc tgtgatatat    1140

gaggcccagg acgtctactc aggtgacatc atcagtggcc tccgagatga aaccaacttc    1200

acagtgatca tcaacccttc aggggtagtg atgtggtacc tgtatcccat caagaacctg    1260

gagatgtccc agcagtga                                                  1278

<210>  8
<211>  425
<212>  PRT
<213>  Artificial

<220>
<223>  recombinant protein

<400>  8

Met Gln Leu Arg Asn Pro Glu Leu His Leu Gly Cys Ala Leu Ala Leu
1               5                   10                  15

Arg Phe Leu Ala Leu Val Ser Trp Asp Ile Pro Gly Ala Arg Ala Leu
            20                  25                  30

Asp Asn Gly Leu Leu Gln Thr Pro Pro Met Gly Trp Leu Ala Trp Glu
        35                  40                  45

Arg Phe Arg Cys Asn Ile Asn Cys Asp Glu Asp Pro Lys Asn Cys Ile
    50                  55                  60

Ser Glu Gln Leu Phe Met Glu Met Ala Asp Arg Met Ala Gln Asp Gly
65                  70                  75                  80

Trp Arg Asp Met Gly Tyr Thr Tyr Leu Asn Ile Asp Asp Cys Trp Ile
                85                  90                  95

Gly Gly Arg Asp Ala Ser Gly Arg Leu Met Pro Asp Pro Lys Arg Phe
            100                 105                 110

Pro His Gly Ile Pro Phe Leu Ala Asp Tyr Val His Ser Leu Gly Leu
        115                 120                 125

Lys Leu Gly Ile Tyr Ala Asp Met Gly Asn Phe Thr Cys Met Gly Tyr
    130                 135                 140

Pro Gly Thr Thr Leu Asp Lys Val Val Gln Asp Ala Gln Thr Phe Ala
145                 150                 155                 160

Glu Trp Lys Val Asp Met Leu Lys Leu Asp Gly Cys Phe Ser Thr Pro
                165                 170                 175

Glu Glu Arg Ala Gln Gly Tyr Pro Lys Met Ala Ala Ala Leu Asn Ala
            180                 185                 190

Thr Gly Arg Pro Ile Ala Phe Ser Cys Glu Trp Pro Leu Tyr Glu Gly
        195                 200                 205

Gly Leu Pro Pro Arg Val Asn Tyr Ser Leu Leu Ala Asp Ile Cys Asn
    210                 215                 220

Leu Trp Arg Asn Tyr Asp Asp Ile Gln Asp Ser Trp Trp Ser Val Leu
225                 230                 235                 240

Ser Ile Leu Asn Trp Phe Val Glu His Gln Asp Ile Leu Gln Pro Val
                245                 250                 255

Ala Gly Pro Gly His Trp Asn Asp Pro Asp Met Leu Leu Ile Gly Asn
            260                 265                 270

Phe Gly Leu Ser Leu Glu Gln Ser Arg Ala Gln Met Ala Leu Trp Thr
        275                 280                 285

Val Leu Ala Ala Pro Leu Leu Met Ser Thr Asp Leu Arg Thr Ile Ser
    290                 295                 300

Ala Gln Asn Met Asp Ile Leu Gln Asn Pro Leu Met Ile Lys Ile Asn
305                 310                 315                 320

Gln Asp Pro Leu Gly Ile Gln Gly Arg Arg Ile His Lys Glu Lys Ser
                325                 330                 335

Leu Ile Glu Val Tyr Met Arg Pro Leu Ser Asn Lys Ala Ser Ala Leu
            340                 345                 350

Val Phe Phe Ser Cys Arg Thr Asp Met Pro Tyr Arg Tyr His Ser Ser
        355                 360                 365

Leu Gly Gln Leu Asn Phe Thr Gly Ser Val Ile Tyr Glu Ala Gln Asp
    370                 375                 380

Val Tyr Ser Gly Asp Ile Ile Ser Gly Leu Arg Asp Glu Thr Asn Phe
385                 390                 395                 400

Thr Val Ile Ile Asn Pro Ser Gly Val Val Met Trp Tyr Leu Tyr Pro
                405                 410                 415

Ile Lys Asn Leu Glu Met Ser Gln Gln
            420                 425

<210>  9
<211>  1290
<212>  DNA
<213>  Homo sapiens

<400>  9

atgcagctga ggaacccaga actacatctg ggctgcgcgc ttgcgcttcg cttcctggcc 60

ctcgtttcct gggacatccc tggggctaga gcactggaca atggattggc aaggacgcct 120

accatgggct ggctgcactg ggagcgcttc atgtgcaacc ttgactgcca ggaagagcca 180

gattcctgca tcagtgagaa gctcttcatg gagatggcag agctcatggt ctcagaaggc 240

tggaaggatg caggttatga gtacctctgc attgatgact gttggatggc tccccaaaga 300

gattcagaag gcagacttca ggcagaccct cagcgctttc ctcatgggat tcgccagcta 360

gctaattatg ttcacagcaa aggactgaag ctagggattt atgcagatgt tggaaataaa 420

acctgcgcag gcttccctgg gagttttgga tactacgaca ttgatgccca gacctttgct 480

gactggggag tagatctgct aaaatttgat ggttgttact gtgacagttt ggaaaatttg 540

gcagatggtt ataagcacat gtccttggcc ctgaatagga ctggcagaag cattgtgtac 600

tcctgtgagt ggcctcttta tatgtggccc tttcaaaagc ccaattatac agaaatccga 660

cagtactgca atcactggcg aaattttgct gacattgatg attcctggaa aagtataaag 720

agtatcttgg actggacatc ttttaaccag gagagaattg ttgatgttgc tggaccaggg 780

ggttggaatg acccagatat gttagtgatt ggcaactttg gcctcagctg gaatcagcaa 840

gtaactcaga tggccctctg ggctatcatg gctgctcctt tattcatgtc taatgacctc 900

cgacacatca gccctcaagc caaagctctc cttcaggata aggacgtaat tgccatcaat 960

caggacccct tgggcaagca agggtaccag cttagacagg gagacaactt tgaagtgtgg 1020

gaacgacctc tctcaggctt agcctgggct gtagctatga taaaccggca ggagattggt 1080

ggacctcgct cttataccat cgcagttgct tccctgggta aaggagtggc ctgtaatcct 1140

gcctgcttca tcacacagct cctccctgtg aaaaggaagc tagggttcta tgaatggact 1200

tcaaggttaa gaagtcacat aaatcccaca ggcactgttt tgcttcagct agaaaataca 1260

atgcagatgt cattaaaaga cttactttaa 1290

<210> 10
<211> 429
<212> PRT
<213> Homo sapiens

<400> 10

<210>  11
<211>  45
<212>  DNA
<213>  Homo sapiens

<400>  11
atgtctgcac ttctgatcct agctcttgtt ggagctgcag ttgct                        45

<210>  12
<211>  15
<212>  PRT
<213>  Homo sapiens

<400>  12

Met Ser Ala Leu Leu Ile Leu Ala Leu Val Gly Ala Ala Val Ala
1               5                   10                  15